# EUROPEAN PATENT APPLICATION

(11) **EP 4 375 265 A1**
(43) Date of publication of application: **29.05.2024**
(21) Application number: 23820128.9
(22) Date of filing: 09.06.2023
(51) Int. Cl.: C07C 13/573, C07D 307/91, C07D 407/10, H10K 85/60, H10K 50/11

(54) **NOVEL COMPOUND AND ORGANIC LIGHT EMITTING DEVICE USING SAME**

(30) Priority: 10.06.2022 KR 20220070957
(71) Applicant: LG Chem, Ltd., Yeongdeungpo-gu Seoul 07336 (KR)
(72) Inventor: KIM, Young Kwang, Daejeon 34122 (KR); LEE, Jaechol, Daejeon 34122 (KR); KIM, Yongwook, Daejeon 34122 (KR); KIM, Juhwan, Daejeon 34122 (KR); PARK, Hyungil, Daejeon 34122 (KR); LIM, Byeong Yun, Daejeon 34122 (KR); CHOI, Hyunju, Daejeon 34122 (KR)
(74) Representative: Hoffmann Eitle
(86) International application number: PCT/KR2023/007912
(87) International publication number: WO 2023/239193

(57) **Abstract**

The present disclosure relates to a novel compound and an organic light emitting device comprising the same.

## Description

### [TECHNICAL FIELD]

### CROSS-REFERENCE TO RELATED APPLICATION(S)

This application claims priority benefit of Korean Patent Application No. 10-2022-0070957 filed on June 10, 2022 in the Korean Intellectual Property Office, the content of which is incorporated herein by reference in its entirety.

The present disclosure relates to a novel compound and an organic light emitting device comprising the same.

### [BACKGROUND ART]

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, an excellent luminance, driving voltage and response speed, and thus many studies have proceeded.

The organic light emitting device generally has a structure which comprises an anode, a cathode, and an organic material layer interposed between the anode and the cathode. The organic material layer frequently has a multilayered structure that comprises different materials in order to enhance efficiency and stability of the organic light emitting device, and for example, the organic material layer may be formed of a hole injection layer, a hole transport layer, a light emitting layer, an electron transport layer, an electron injection layer, and the like. In the structure of the organic light emitting device, if a voltage is applied between two electrodes, the holes are injected from an anode into the organic material layer and the electrons are injected from the cathode into the organic material layer, and when the injected holes and electrons meet each other, an exciton is formed, and light is emitted when the exciton falls to a ground state again.

There is a continuous need to develop a new material for the organic material used in the organic light emitting device as described above.

Meanwhile, recently, in order to reduce process costs, an organic light emitting device using a solution process, particularly an inkjet process, has been developed instead of a conventional deposition process. In the initial stage of development, attempts have been made to develop organic light emitting devices by coating all organic light emitting device layers by a solution process, but current technology has limitations. Therefore, only HIL, HTL, and EML are processed by a solution process, and a hybrid process utilizing traditional deposition processes is being studied as a subsequent process.

Therefore, the present disclosure provides a novel material for organic light emitting devices that can be used for an organic light emitting device, and at the same time, can be used in a solution process.

### [Prior Art Literature]

### [Patent Literature]

(Patent Literature 0001) Korean Unexamined Patent Publication No. 10-2000-0051826

### [DETAILED DESCRIPTION OF THE INVENTION]

### [Technical Problem]

It is an object of the present disclosure to provide a novel compound and an organic light emitting device including the same.

### [Technical Solution]

Provided herein is a compound represented by the following Chemical Formula 1: 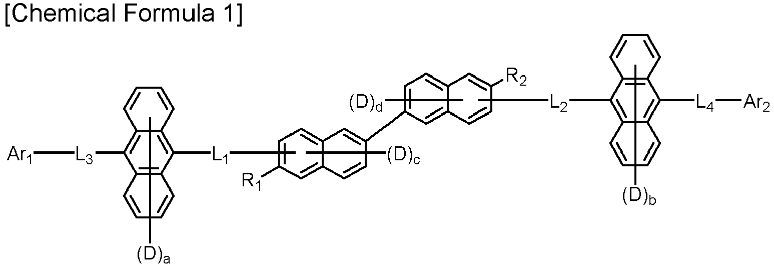 wherein, in Chemical Formula 1,
D means deuterium,
R₁ and R₂ each independently represent hydrogen or deuterium;
L₁ to L₄ each independently represent a single bond; or a substituted or unsubstituted C₆₋₆₀ arylene,
Ar₁ and Ar₂ each independently represent a substituted or unsubstituted C₆₋₆₀ aryl; or a substituted or unsubstituted C₂₋₆₀ heteroaryl containing one or more heteroatoms of N, O and S,
a and b are each independently an integer of 0 to 8, and
c and d are each independently an integer of 0 to 5.

Also provided herein is an organic light emitting device comprising: a first electrode; a second electrode that is provided opposite to the first electrode; and one or more organic material layers that are provided between the first electrode and the second electrode, wherein the one or more layers of the organic material layers comprises the compound represented by Chemical Formula 1.

### [ADVANTAGEOUS EFFECTS]

The compound represented by Chemical Formula 1 as mentioned above can be used as a material of an organic material layer in an organic light emitting device, can be used in a solution process, and can improve the efficiency and lifetime characteristics in the organic light emitting device.

### [BRIEF DESCRIPTION OF THE DRAWINGS]

FIG. 1 shows an example of an organic light emitting device comprising a substrate 1, an anode 2, a light emitting layer 3, and a cathode 4.
FIG. 2 shows an example of an organic light emitting device comprising a substrate 1, an anode 2, a hole injection layer 5, a hole transport layer 6, a light emitting layer 3, an electron injection and transport layer 7, and a cathode 4.

### [DETAILED DESCRIPTION OF THE EMBODIMENTS]

Hereinafter, embodiments of the present disclosure will be described for a more complete understanding of the disclosed subject matter.

### (Definition of Terms]

As used herein, the term "substituted or unsubstituted" means being unsubstituted or substituted with one or more substituents selected from the group consisting of deuterium; a halogen group; a cyano group; a nitro group; a hydroxy group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; or a heterocyclic group containing one or more of N, O and S atoms, or being unsubstituted or substituted with a substituent to which two or more substituents of the above-exemplified substituents are linked. For example, "a substituent in which two or more substituents are linked" may be a biphenyl group. Namely, a biphenylyl group may be an aryl group, or it may also be interpreted as a substituent in which two phenyl groups are linked. In one example, the term "substituted or unsubstituted" may be understood to mean "being unsubstituted or substituted with one or more substituents selected from the group consisting of deuterium, halogen, cyano, a C₁₋₁₀ alkyl, a C₁₋₁₀ alkoxy and a C₆₋₂₀ aryl", or "being unsubstituted or substituted with one or more substituents selected from the group consisting of deuterium, halogen, cyano, methyl, ethyl, phenyl and naphthyl". Further, the term "substituted with one or more substituents" as used herein may be understood to mean "being substituted with mono to the maximum number of available substitutable hydrogen atoms". Alternatively, the term "substituted with one or more substituents" as used herein may be understood to mean "being substituted with 1 to 5 substituents", or "being substituted with one or two substituents".

In the present disclosure, the carbon number of a carbonyl group is not particularly limited, but is preferably 1 to 40. Specifically, it may be a substituent group having the following structure, but is not limited thereto.

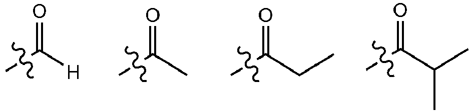

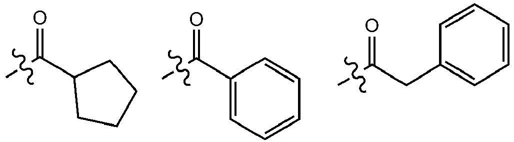

In the present disclosure, an ester group may have a structure in which oxygen of the ester group may be substituted by a straight-chain, branched-chain, or cyclic alkyl group having 1 to 25 carbon atoms, or an aryl group having 6 to 25 carbon atoms. Specifically, the ester group may be a substituent group having the following structural formulas, but is not limited thereto.

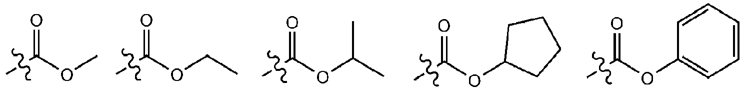

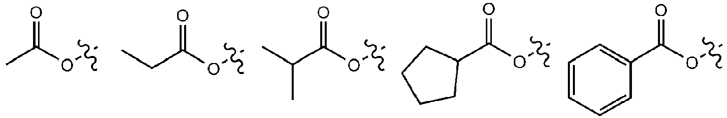

In the present disclosure, the carbon number of an imide group is not particularly limited, but is preferably 1 to 25. Specifically, the imide group may be a substituent group having the following structural formulas, but is not limited thereto.

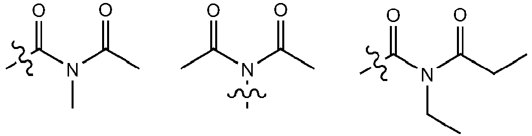

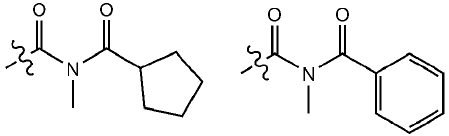

In the present disclosure, a substituted or unsubstituted silyl group refers to -Si(Z₁)(Z₂)(Z₃), wherein Z₁, Z₂ and Z₃ may each independently represent hydrogen, deuterium, a substituted or unsubstituted C₁₋₆₀ alkyl, a substituted or unsubstituted C₁₋₆₀ haloalkyl, a substituted or unsubstituted C₂₋₆₀ alkenyl, a substituted or unsubstituted C₂₋₆₀ haloalkenyl, or a substituted or unsubstituted C₆₋₆₀ aryl. According to one embodiment, Z₁, Z₂ and Z₃ may each independently represent hydrogen, deuterium, a substituted or unsubstituted C₁₋₁₀ alkyl, a substituted or unsubstituted C₁₋₁₀ haloalkyl, a substituted or unsubstituted C₁₋₁₀ haloalkyl, or a substituted or unsubstituted C₆₋₂₀ aryl. Specific examples of the silyl group include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but is not limited thereto.

In the present disclosure, a boron group specifically includes a trimethylboron group, a triethylboron group, a t-butyldimethylboron group, a triphenylboron group, a phenylboron group, and the like, but is not limited thereto.

In the present disclosure, examples of a halogen group include fluorine, chlorine, bromine, or iodine.

In the present disclosure, the alkyl group may be straight-chain or branched-chain, and the carbon number thereof is not particularly limited, but is preferably 1 to 40. According to one embodiment, the carbon number of the alkyl group is 1 to 20. According to another embodiment, the carbon number of the alkyl group is 1 to 10. According to another embodiment, specific examples of the alkyl group include methyl, ethyl, propyl, n-propyl, isopropyl, butyl, n-butyl, isobutyl, tert-butyl, sec-butyl, 1-methylbutyl, 1-ethylbutyl, pentyl, n-pentyl, isopentyl, neopentyl, tert-pentyl, 1-ethylpropyl, 1,1-dimethylpropyl, hexyl, n-hexyl, 1-methylpentyl, 2-methylpentyl, 4-methyl-2-pentyl, 3,3-dimethylbutyl, 2-ethylbutyl, heptyl, n-heptyl, isohexyl, 1-methylhexyl, 2-methylhexyl, 3-methylhexyl, 4-methylhexyl, 5-methylhexyl, cyclopentylmethyl, cyclohexylmethyl, octyl, n-octyl, tert-octyl, 1-methylheptyl, 2-ethylhexyl, 2,4,4-trimethyl-1-pentyl, 2,4,4-trimethyl-2-pentyl, 2-propylpentyl, n-nonyl, 2,2-dimethylheptyl, and the like, but are not limited thereto.

In the present disclosure, the alkenyl group may be straight-chain or branched-chain, and the carbon number thereof is not particularly limited, but is preferably 2 to 40. According to one embodiment, the carbon number of the alkenyl group is 2 to 20. According to another embodiment, the carbon number of the alkenyl group is 2 to 10. According to still another embodiment, the carbon number of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present disclosure, a cycloalkyl group is not particularly limited, but the carbon number thereof is preferably 3 to 60. According to one embodiment, the carbon number of the cycloalkyl group is 3 to 30. According to another embodiment, the carbon number of the cycloalkyl group is 3 to 20. According to still another embodiment, the carbon number of the cycloalkyl group is 3 to 6. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present disclosure, an aryl group is understood to mean a substituent group derived from a monocyclic or fused polycyclic compound having aromaticity while containing only a carbon atom as a ring-constituting atom, and the carbon number thereof is not particularly limited, but is preferably 6 to 60. According to one embodiment, the carbon number of the aryl group is 6 to 30. According to one embodiment, the carbon number of the aryl group is 6 to 20. The aryl group may be a phenyl group, a biphenylyl group, a terphenylyl group or the like as the monocyclic aryl group, but is not limited thereto. The polycyclic aryl group includes a naphthyl group, an anthracenyl group, a phenanthrenyl group, a pyrenyl group, a perylenyl group, a chrysenyl group, or the like, but is not limited thereto.

In the present disclosure, the fluorenyl group may be substituted, and two substituent groups may be linked with each other to form a spiro structure. In the case where the fluorenyl group is substituted, 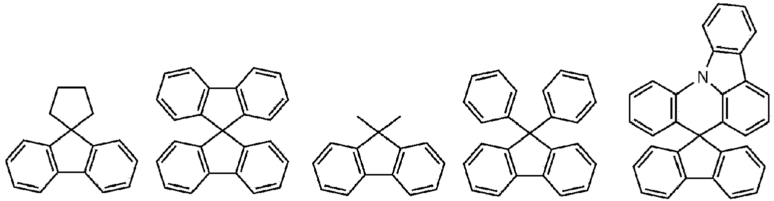 and the like can be formed. However, the structure is not limited thereto.

In the present disclosure, a heteroaryl group refers to a substituent group derived from a monocyclic or fused polycyclic compound further containing at least one heteroatom selected from N, O and S in addition to a carbon atom as a ring-constituting atom, and means a substituent group having aromaticity. According to one embodiment, the carbon number of the heteroaryl group is 2 to 60. According to another embodiment, the carbon number of the heteroaryl group is 2 to 30. According to another embodiment, the carbon number of the heteroaryl group is 2 to 20. Examples of the heteroaryl group include a thiophenyl group, a furanyl group, a pyrrolyl group, an imidazolyl group, a thiazolyl group, an oxazolyl group, an oxadiazolyl group, a triazolyl group, a pyridinyl group, a bipyridinyl group, a pyrimidinyl group, a triazinyl group, an acridinyl group, a pyridazinyl group, a pyrazinyl group, a quinolinyl group, a quinazolinyl group, a quinoxalinyl group, a phthalazinyl group, a pyridopyrimidinyl group, a pyridopyrazinyl group, an isoquinolinyl group, an indolyl group, a carbazolyl group, a benzoxazolyl group, a benzoimidazolyl group, a benzothiazolyl group, a benzocarbazolyl group, a benzothiophenyl group, a dibenzothiophenyl group, a benzofuranyl group, a dibenzofuranyl group, a phenanthrolinyl group, an isoxazolyl group, a thiadiazolyl group, a phenothiazinyl group, and the like, but are not limited thereto.

In the present disclosure, the aryl group in the aralkyl group, the aralkenyl group, the alkylaryl group, the arylamine group and the arylsilyl group is the same as the examples of the aryl group as defined above. In the present disclosure, the alkyl group in the aralkyl group, the alkylaryl group and the alkylamine group is the same as the examples of the alkyl group as defined above. In the present disclosure, the heteroaryl in the heteroarylamine can be applied to the description of the heteroaryl as defined above. In the present disclosure, the alkenyl group in the aralkenyl group is the same as the examples of the alkenyl group as defined above. In the present disclosure, the description of the aryl group as defined above may be applied except that the arylene is a divalent group. In the present disclosure, the description of the heteroaryl as defined above can be applied except that the heteroarylene is a divalent group. In the present disclosure, the description of the aryl group or cycloalkyl group as defined above can be applied except that the hydrocarbon ring is not a monovalent group but formed by combining two substituent groups. In the present disclosure, the description of the heteroaryl as defined above can be applied, except that the heterocycle is not a monovalent group but formed by combining two substituent groups.

In the present disclosure, the term "deuterated or substituted with deuterium" means that at least one of the substitutable hydrogens in a compound, a divalent linking group, or a monovalent substituent has been substituted with deuterium.

Further, the term "unsubstituted or substituted with deuterium" or "substituted or unsubstituted with deuterium" means that "mono to the maximum number of unsubstituted or substitutable hydrogens have been substituted with deuterium." In one example, the term "phenanthryl unsubstituted or substituted with deuterium" may be understood to mean "phenanthryl unsubstituted or substituted with 1 to 9 deuterium atoms", considering that the maximum number of hydrogen that can be substituted with deuterium in the phenanthryl structure is 9.

Further, the term "deuterated structure" is meant to encompass a compound, a divalent linking group or a monovalent substituent of all structures in which at least one hydrogen is replaced with deuterium. In one example, a deuterated structure of phenyl may be understood to refer to monovalent substituents of all structures in which at least one substitutable hydrogen in a phenyl group is replaced with a deuterium as follows.

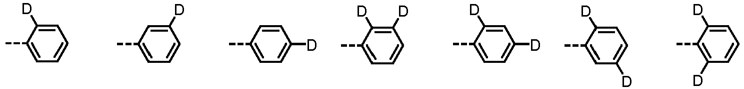

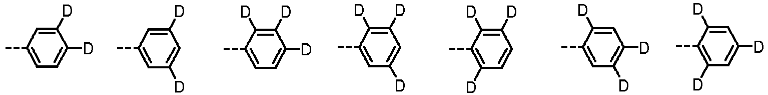

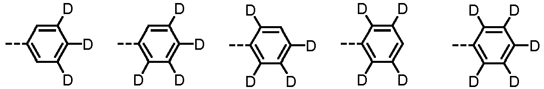

In addition, the "deuterium substitution rate" or "degree of deuteration" of a compound means that the ratio of the number of substituted deuterium atoms to the total number of hydrogen atoms (the sum of the number of hydrogen atoms substitutable with deuterium and the number of substituted deuterium atoms in a compound) that can be present in the compound is calculated as a percentage. Therefore, when the "deuterium substitution rate" or "degree of deuteration" of a compound is "K%", it means that K% of the hydrogen atoms substitutable with deuterium in the compound are substituted with deuterium.

At this time, the "deuterium substitution rate" or "degree of deuteration" can be measured according to a commonly known method using MALDI-TOF MS (Matrix-Assisted Laser Desorption/Ionization Time-of-Flight Mass Spectrometer), a nuclear magnetic resonance spectroscopy (¹H NMR), TLC/MS (Thin-Layer Chromatography/Mass Spectrometry), GC/MS (Gas Chromatography/Mass Spectrometry), or the like. More specifically, when using MALDI-TOF MS, the "deuterium substitution rate" or "degree of deuteration" is obtained by determining the number of substituted deuterium atoms in the compound through MALDI-TOF MS analysis, and then calculating the ratio of the number of substituted deuterium atoms to the total number of hydrogen atoms that can be present in the compound as a percentage.

### (Compound)

According to the present disclosure, there is provided the compound represented by Chemical Formula 1.

An anthracene-based compound, which has been conventionally used as a host material, is low molecular weight compounds having a molecular weight of 400 to 600 g/mol, which is excellent in solubility in a solvent, and thus, can be used to form a light emitting layer in a solution process. However, when a separate layer such as an electron transport layer is subsequently applied on the light emitting layer by a solution process, there was problem that resistance to a solvent for forming the electron transport layer is not good.

Thereby, there has been a demand for organic materials that exhibits high solubility in the solvent for forming the light emitting layer and at the same time, can exhibit resistance to the solvent for forming the hole blocking layer, the electron transport layer or the electron injection and transport layer, etc., which can be provided on the light emitting layer.

Thus, the present inventors have found that from the view point of molecular orbitals, in the case of a compound containing two anthracene structures while having a (naphthylene)-(naphthylene) linker, if naphthylenes are linked to each other at positions 2 and 2', and anthracene is linked to any one of positions 5, 7 and 8 and any one of positions 5', 7' and 8', the compound can exhibit high solubility in the solvent for forming the light emitting layer while having a molecular weight sufficient to exhibit resistance to the solvent for forming a separate layer on the light emitting layer, and completed the present disclosure.

The compound represented by Chemical Formula 1 is a compound having a structure in which two anthracene rings are linked by a binaphthylene linker, and the binapthylene linker has the feature that naphthylenes are linked to each other at positions 2 and 2', and anthracenes are linked to any one of positions 5, 7 and 8 and any one of positions 5', 7' and 8'.

The compound represented by Chemical Formula 1 may exhibit excellent material stability due to its structural features as compared with a compound having a structure in which a binaphthylene linker is linked to two anthracenes at positions 6 and 6', and a compound in which the binaphthylene linker is not linked to each other at positions 2 and 2'.

Specifically, there is a problem that a compound having a molecular weight of a certain level or higher does not dissolve in a solvent used in a solution process due to the increased molecular weight. Therefore, it is necessary to induce a twist form in molecule sufficient to increase its solubility in solvents. However, from the view point of molecular orbitals, in the case of a compound having a (naphthylene)-(naphthylene) linker and containing two anthracene structures, if naphthylenes are linked to each other at the positions 2 and 2', and anthracenes are linked to any one of positions 5, 7 and 8 and any one of positions 5', 7' and 8', a twist form in molecule can be effectively induced.

Thereby, the compound represented by Chemical Formula 1 has a molecular weight of a certain level or higher, and thus, can exhibit resistance to a solvent for forming a separate layer on the light emitting layer, and at the same time, has high solubility as compared to a compound having a binapthylene linker different from Chemical Formula 1, and can effectively form a light emitting layer in a solution process using the compound. Therefore, the efficiency and lifetime of the finally provided organic light emitting device including the compound represented by Chemical Formula 1 as a light emitting layer can be improved.

Specifically, the bonding positions of the binaphthylene linker and L₁ and L₂ in the compound represented by Chemical Formula 1 are as follows: 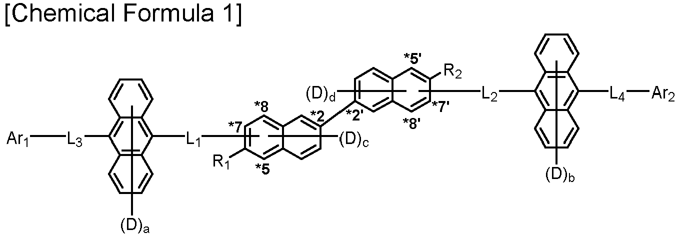 wherein, in Chemical Formula 1,
L₁ binds to any one of carbon at position *5, carbon at position *7, and carbon at position *8 of the binaphthylene linker, and
L₂ binds to any one of carbon at position *5', carbon at position *7', and carbon at position *8' of the binaphthylene linker.

Specifically, when the bonding position between the binaphthylene linker and L₁ and L₂ is represented by (L₁ bonding position, L₂ bonding position), the bonding position of the binaphthylene linker and L₁ and L₂ may be (*5, *5'), (*5, *7'), (*5, *8'), (*7, *7'), (*7, *8'), or (*8, *8').

Among them, those in which the bonding position of the binaphthylene linker and L₁ and L₂ are (*5, *5'), (*7, *7') or (*8, *8') is preferable in terms of ease of synthesis.

In Chemical Formula 1, both R₁ and R₂ may be hydrogen; or deuterium.

Further, L₁ to L₄ may each independently represent a single bond; or a C₆₋₂₀ arylene unsubstituted or substituted with deuterium.

Further, L₁ to L₄ may each independently represent a single bond, a substituted or unsubstituted phenylene, a substituted or unsubstituted biphenyldiyl, or a substituted or unsubstituted naphthylene.

Further, L₁ to L₄ may each independently represent a single bond; 1,2-phenylene unsubstituted or substituted with 1 to 4 deuterium atoms; 1,3-phenylene unsubstituted or substituted with 1 to 4 deuterium atoms; or 1,4-phenylene unsubstituted or substituted with 1 to 4 deuterium atoms.

Specifically, both L₁ and L₂ are a single bond; or
one of L₁ and L₂ may be a single bond, and the remaining one may be phenylene unsubstituted or substituted with 1 to 4 deuterium atoms.

For example, both L₁ and L₂ are a single bond; or
one of L₁ and L₂ is a single bond, and the remaining one may be 1,2-phenylene unsubstituted or substituted with 1 to 4 deuterium atoms; 1,3-phenylene unsubstituted or substituted with 1 to 4 deuterium atoms; or 1,4-phenylene unsubstituted or substituted with 1 to 4 deuterium atoms.

Further, specifically, both L₃ and L₄ are a single bond; or
one of L₃ and L₄ is a single bond, and the remaining one may be phenylene unsubstituted or substituted with 1 to 4 deuterium atoms.

For example, both L₃ and L₄ are a single bond; or
one of L₃ and L₄ is a single bond; the remaining one may be 1,2-phenylene unsubstituted or substituted with 1 to 4 deuterium atoms; 1,3-phenylene unsubstituted or substituted with 1 to 4 deuterium atoms; or 1,4-phenylene unsubstituted or substituted with 1 to 4 deuterium atoms.

Further, L₁ to L₄ are all single bonds; or
L₁ to L₄ may each independently represent 1,2-phenylene unsubstituted or substituted with 1 to 4 deuterium atoms; 1,3-phenylene unsubstituted or substituted with 1 to 4 deuterium atoms; or 1,4-phenylene unsubstituted or substituted with 1 to 4 deuterium atoms, and all the remainder may be a single bond.

Further, L₁ and L₂ may be the same as each other. Alternatively, L₁ and L₂ may be different.

Further, L₃ and L₄ may be the same as each other. Alternatively, L₃ and L₄ may be different.

Further, Ar₁ and Ar₂ may each independently represent a substituted or unsubstituted C₆₋₂₀ aryl; or a substituted or unsubstituted C₂₋₂₀ heteroaryl containing one or two heteroatoms of N, O and S.

Further, Ar₁ and Ar₂ may each independently represent a C₆₋₂₀ aryl which is unsubstituted or substituted with one or more substituents selected from the group consisting of deuterium and phenyl; or a C₂₋₂₀ heteroaryl containing one or two heteroatoms of N, O and S, which is unsubstituted or substituted with one or more substituents selected from the group consisting of deuterium and phenyl.

Specifically, Ar₁ and Ar₂ may each independently represent a substituted or unsubstituted phenyl, a substituted or unsubstituted biphenylyl, a substituted or unsubstituted naphthyl, a substituted or unsubstituted phenanthryl, a substituted or unsubstituted dibenzofuranyl, a substituted or unsubstituted dibenzothiophenyl, a substituted or unsubstituted carbazolyl, or a substituted or unsubstituted phenylcarbazolyl.

More specifically, Ar₁ and Ar₂ each independently represent phenyl, biphenylyl, naphthyl, phenanthryl, dibenzofuranyl, dibenzothiophenyl, carbazolyl, or phenylcarbazolyl,
wherein Ar₁ and Ar₂ may be unsubstituted or substituted with at least one deuterium.

Further, Ar₁ and Ar₂ may be the same as each other. Alternatively, Ar₁ and Ar₂ may be different.

Further, L₃-Ar₁ and L₄-Ar₂ may be the same as each other. Alternatively, L₃-Ar₁ and L₄-Ar₂ may be different.

Further, a and b, which means the number of deuterium, are each independently 0, 1, 2, 3, 4, 5, 6, 7, or 8, and c and d are each independently 0, 1, 2, 3, 4 or 5.

In one embodiment, a+b+c+d may be 0, or 10 or more.

For example, a+b+c+d is 0, or may be 10 or more, 15 or more, 20 or more, 25 or more, 26 or more, 27 or more, or 28 or more, and 36 or less.

Further, the compound may be represented by any one of Chemical Formulas 1-1 to 1-3: 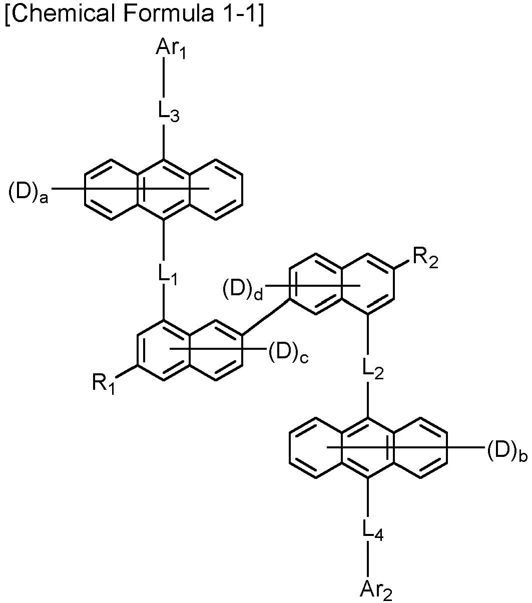 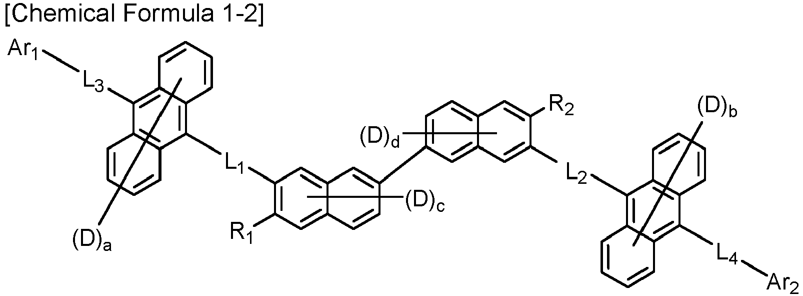 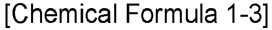 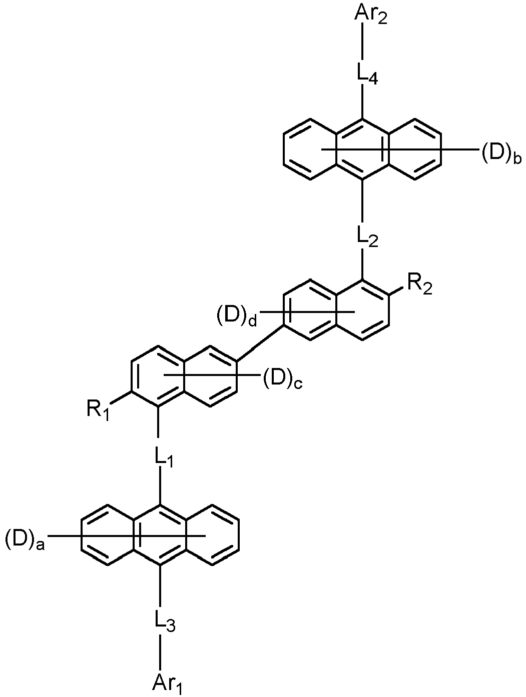 in Chemical Formulas 1-1 to 1-3,
R₁, R₂, L₁ to L₄, Ar₁, Ar₂ and a to d are as defined in Chemical Formula 1.

Further, the compound may not contain deuterium, or may contain at least one deuterium.

Specifically, the compound may not contain deuterium, or may contain 1 or more, 5 or more, 10 or more, 20 or more, 30 or more, 35 or more, or 40 or more, and 60 or less, 55 or less, 50 or less, 49 or less, 48 or less, 47 or less, or 46 or less deuterium atoms.

More specifically, the deuterium substitution rate of the compound may be 0% or 80% or more.

In one example, when the compound contains deuterium, the deuterium substitution rate of the compound may be 1% to 100%. Specifically, the deuterium substitution rate of the compound may be 5% or more, 10% or more, 20% or more, 30% or more, 40% or more, 50% or more, 60% or more, 70% or more, or 80% or more, and 100% or less, 95% or less, or 90% or less.

Further, the compound may have a molecular weight of 800 g/mol or more. More specifically, the compound has a molecular weight (g/mol) of 850 or more, 860 or more, 870 or more, 880 or more, 890 or more, 900 or more, 910 or more, 920 or more, or 930 or more, and 2,000 or less, 1,500 or less, 1,300 or less, 1,200 or less, 1,100 or less, 1,080 or less, 1,060 or less, 1,040 or less, 1,020 or less, or 1,000 or less.

In this case, when the number of deuterium substitution of the compound is to be represented, it can be represented by the following Chemical Formula 1 D: 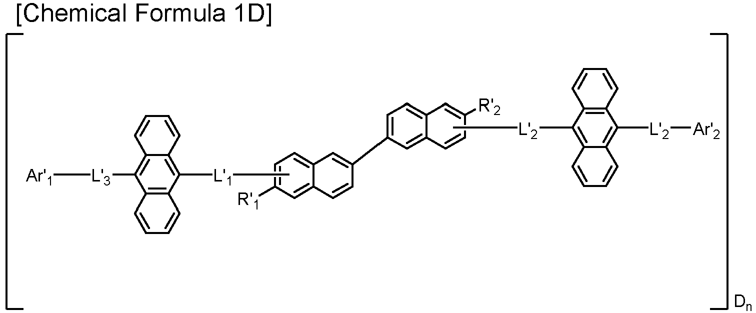 wherein, in Chemical Formula 1D,
Dn means that n hydrogens have been replaced by deuterium,
wherein n is an integer of 0 or more,
R'₁, R'₂, L'₁ to L'₄, Ar'₁ and Ar'₂ mean R₁, R₂, L₁ to L₄, Ar₁ and Ar₂ substituents which are not substituted with deuterium, respectively.

In one embodiment, when the compound has at least one deuterium, n in Chemical Formula 1D is an integer of 1 or more.

On the other hand, representative examples of the compound represented by Chemical Formula 1 is any one selected from the group consisting of compounds represented by the following Chemical Formulas and their deuterated structures:

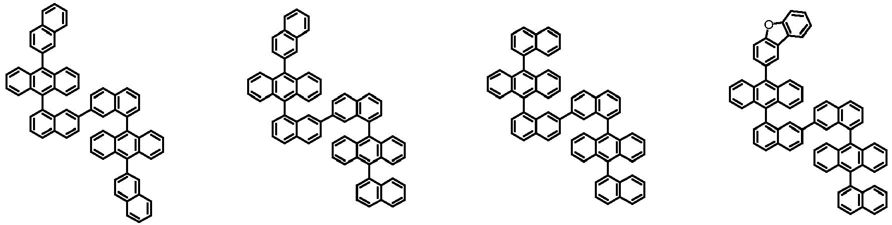

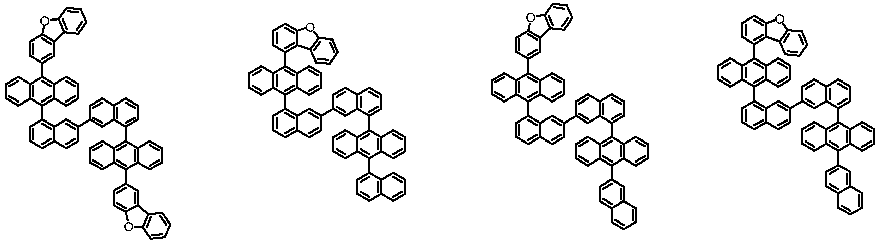

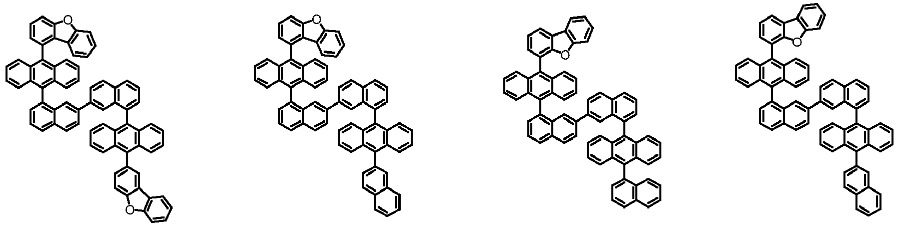

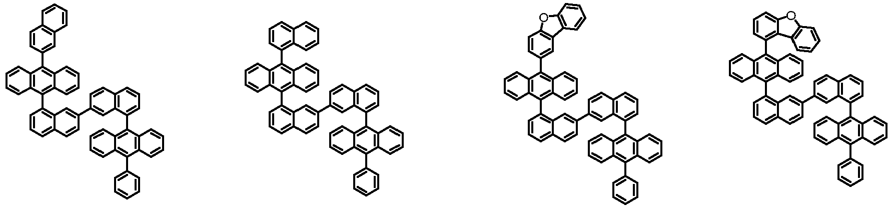

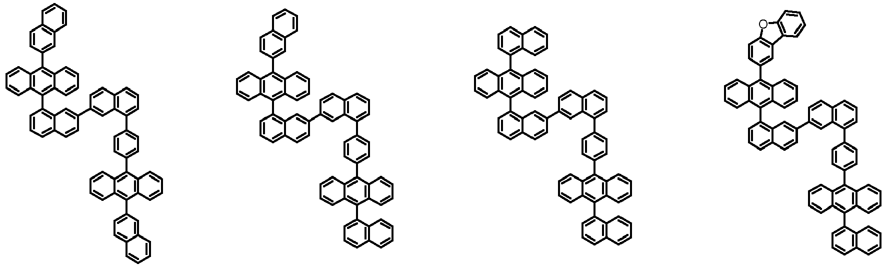

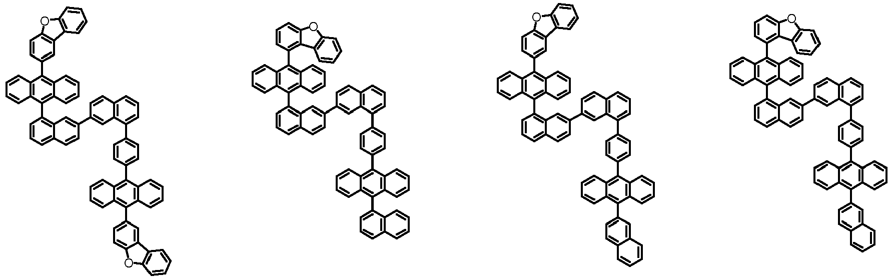

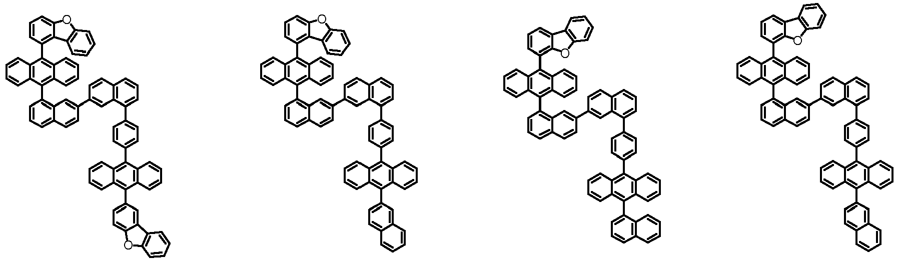

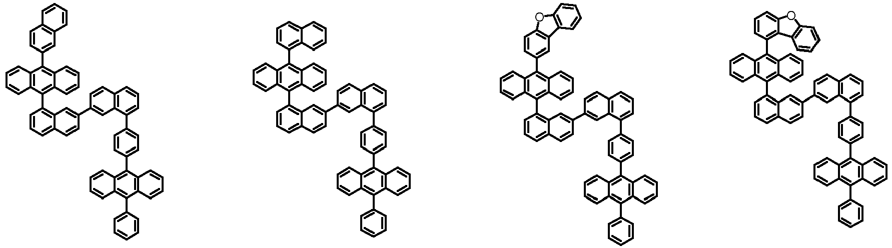

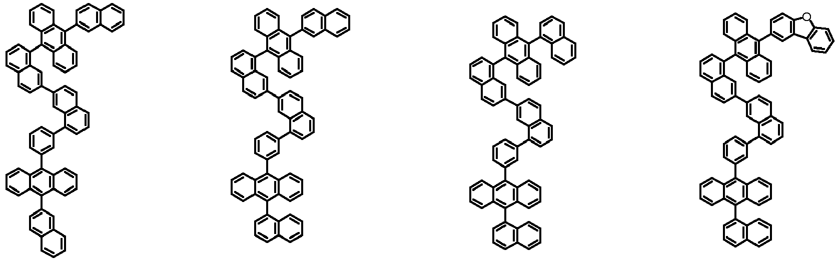

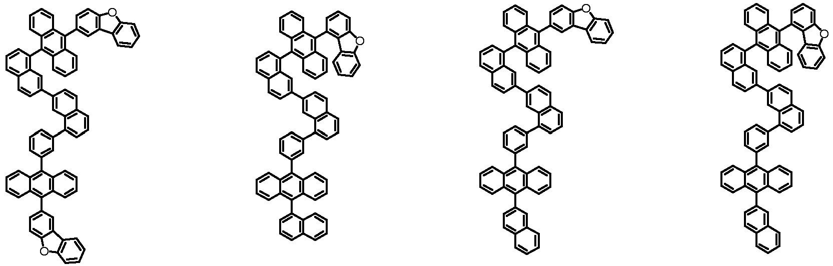

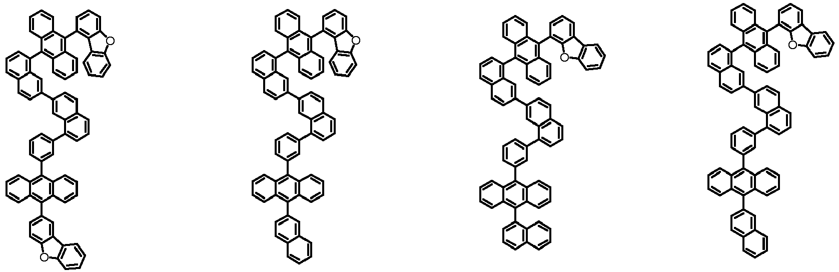

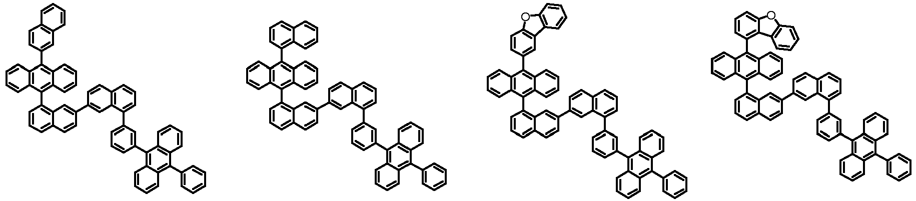

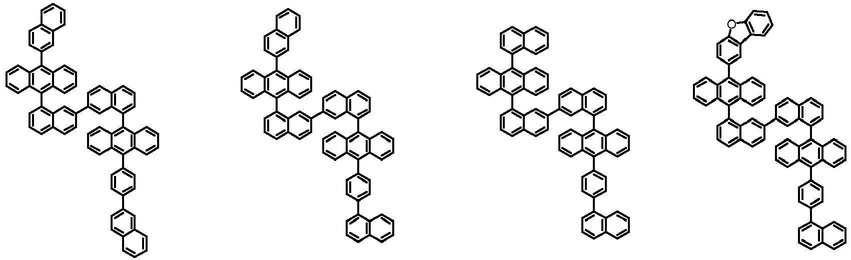

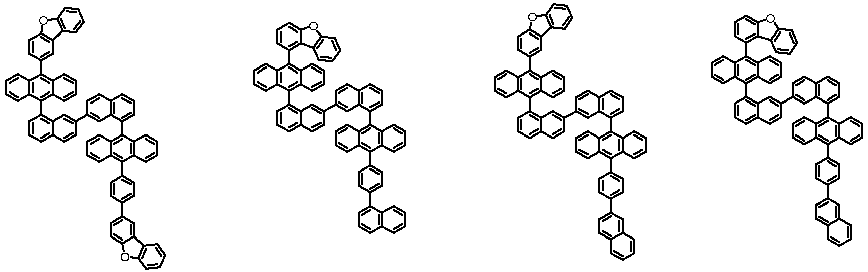

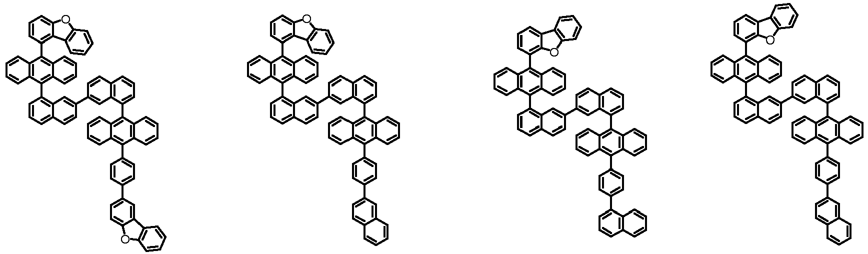

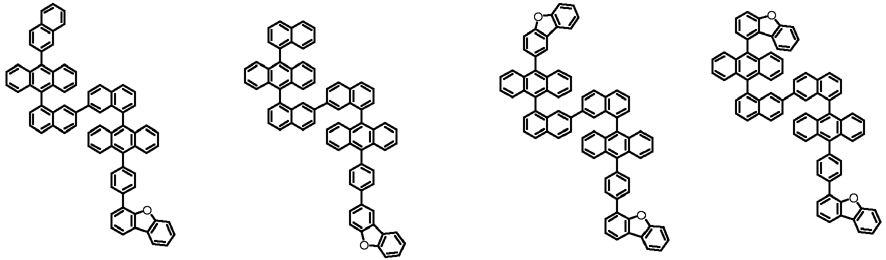

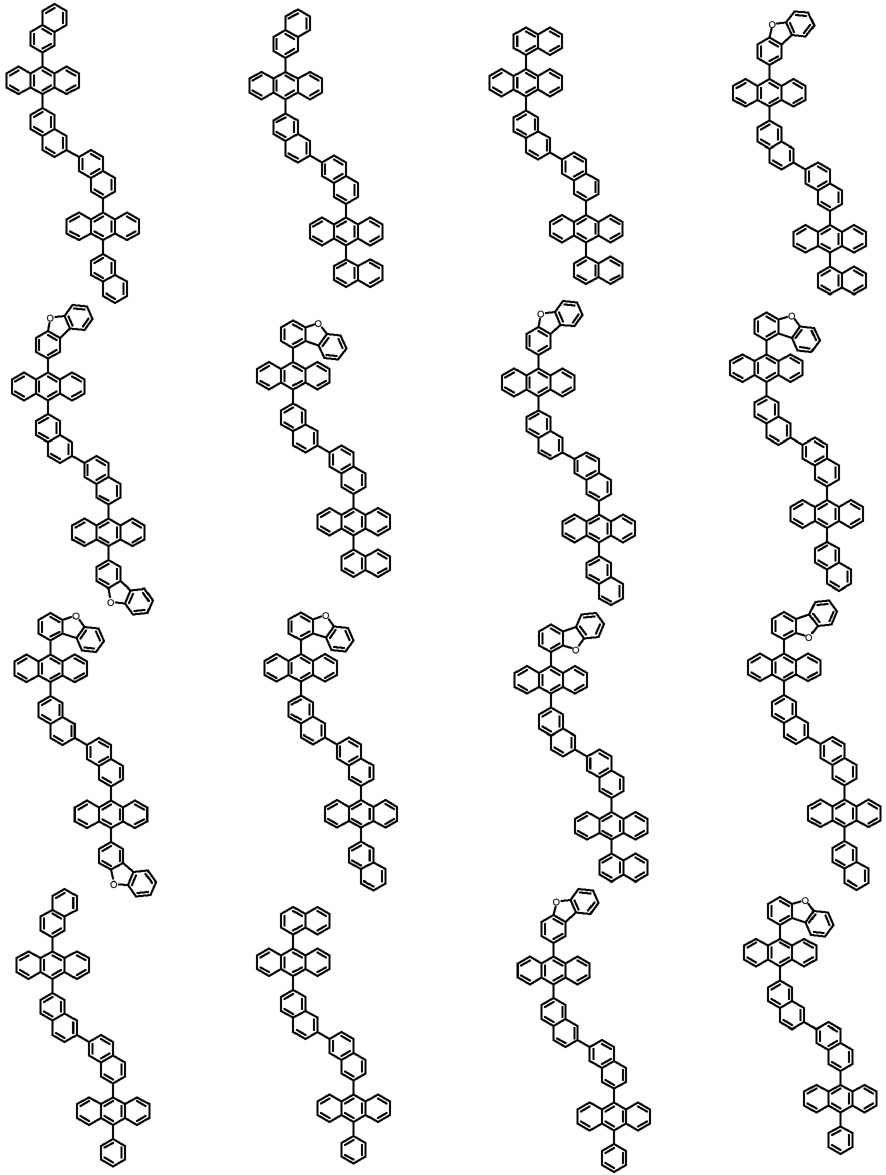

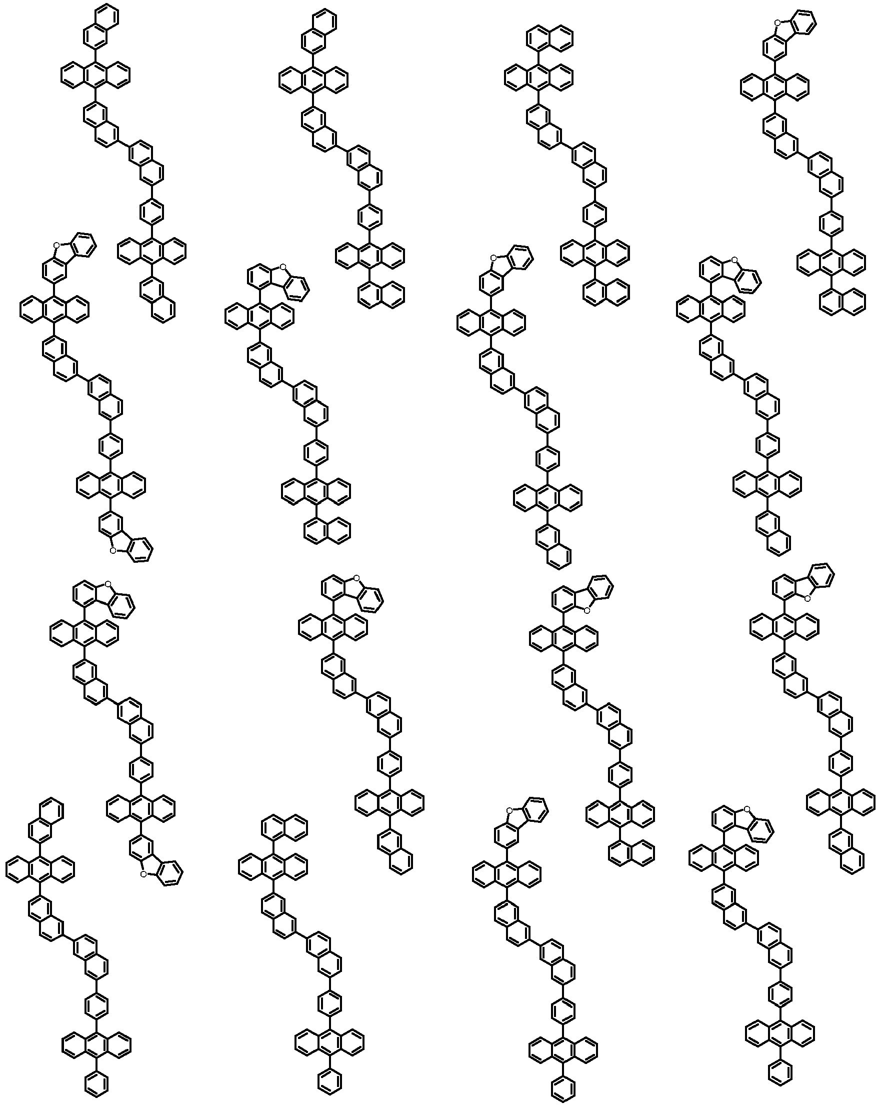

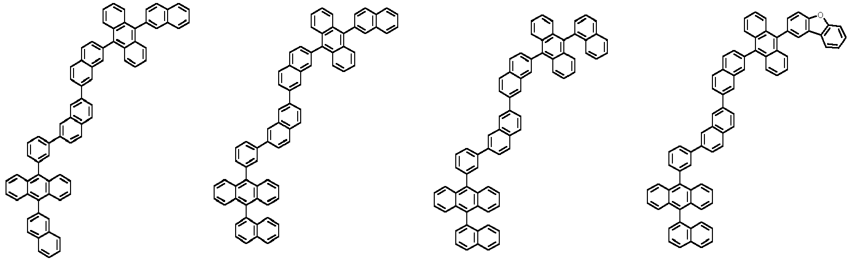

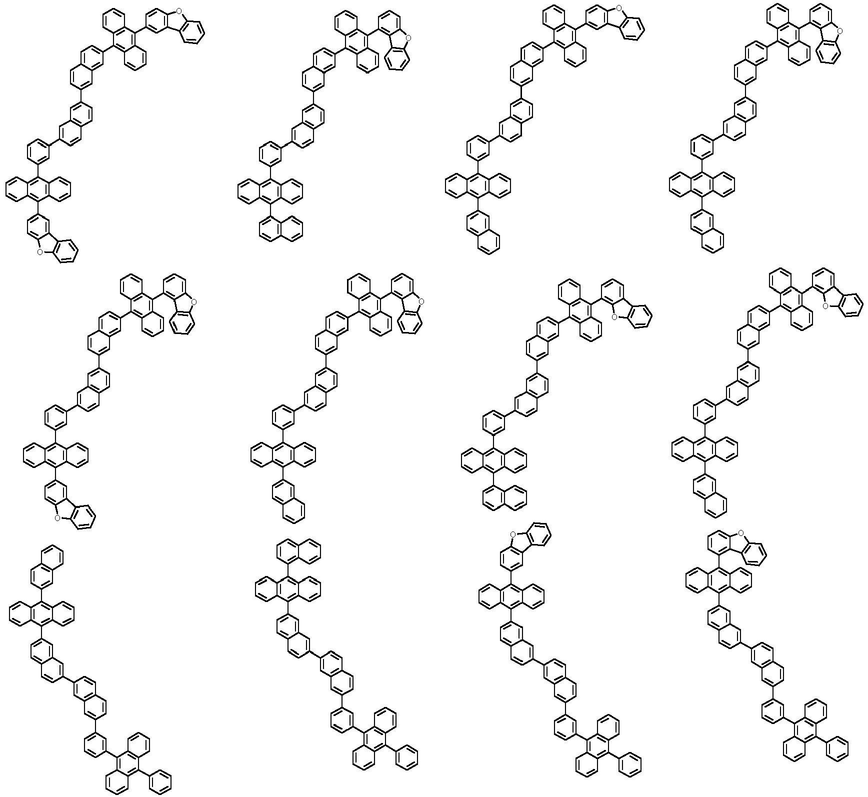

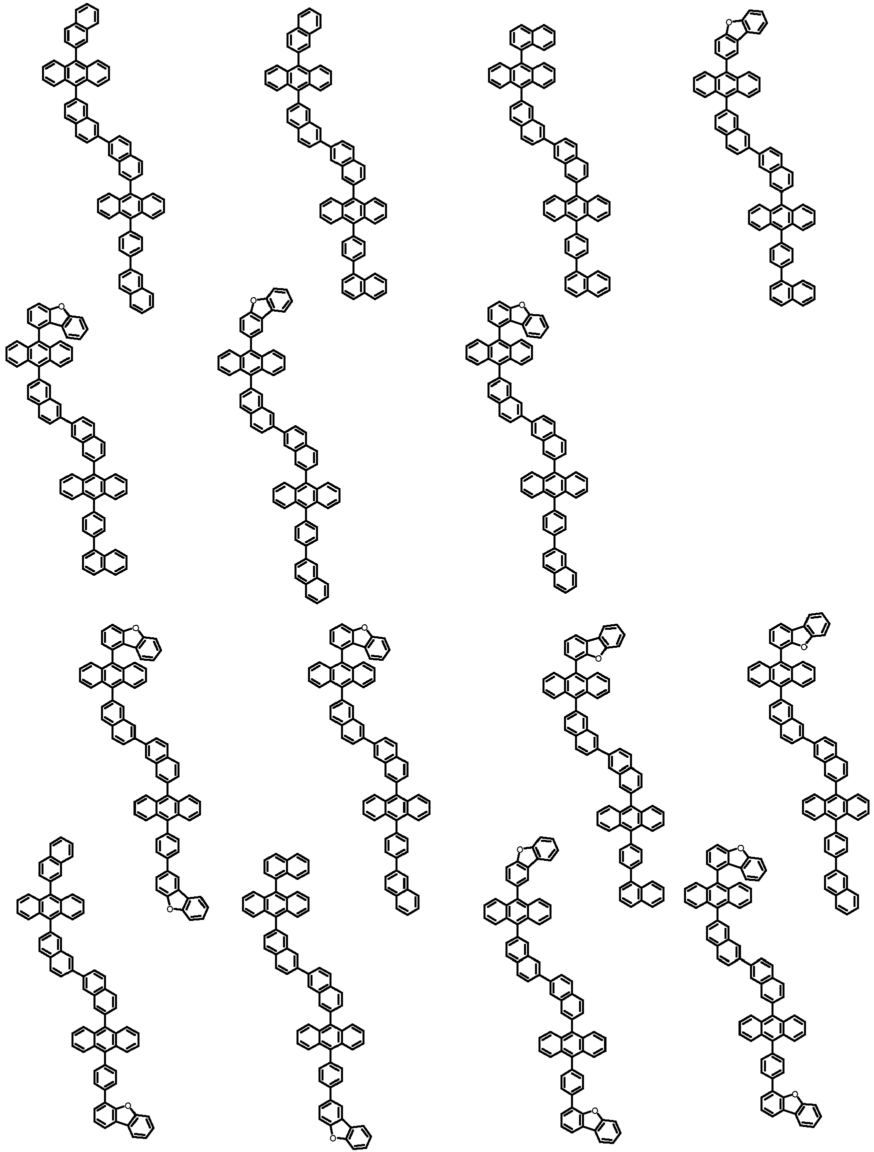

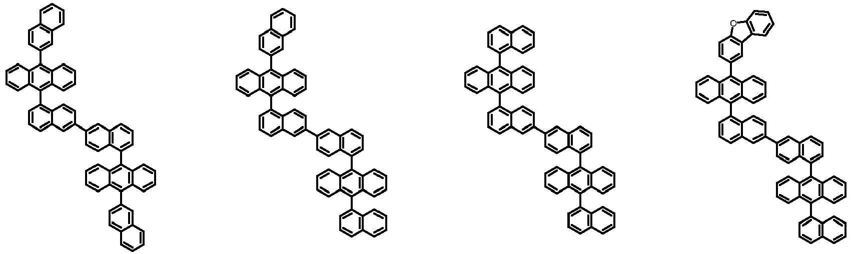

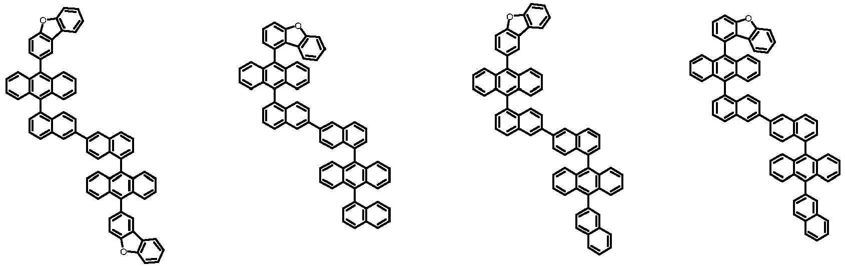

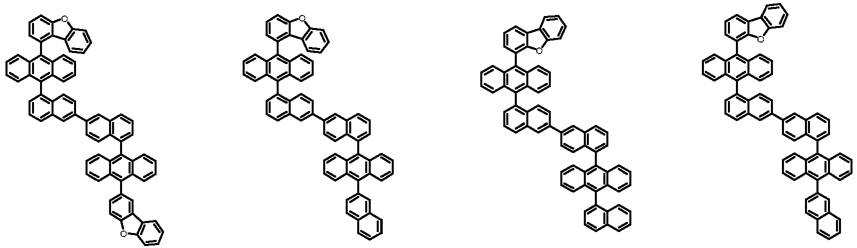

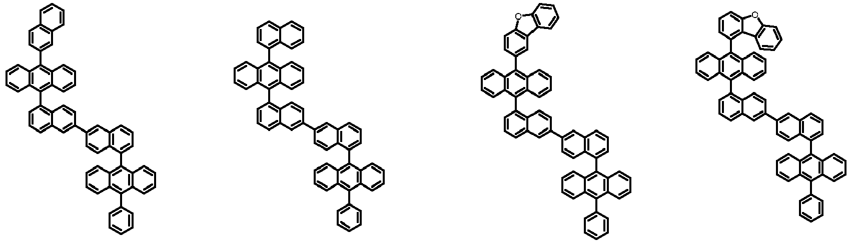

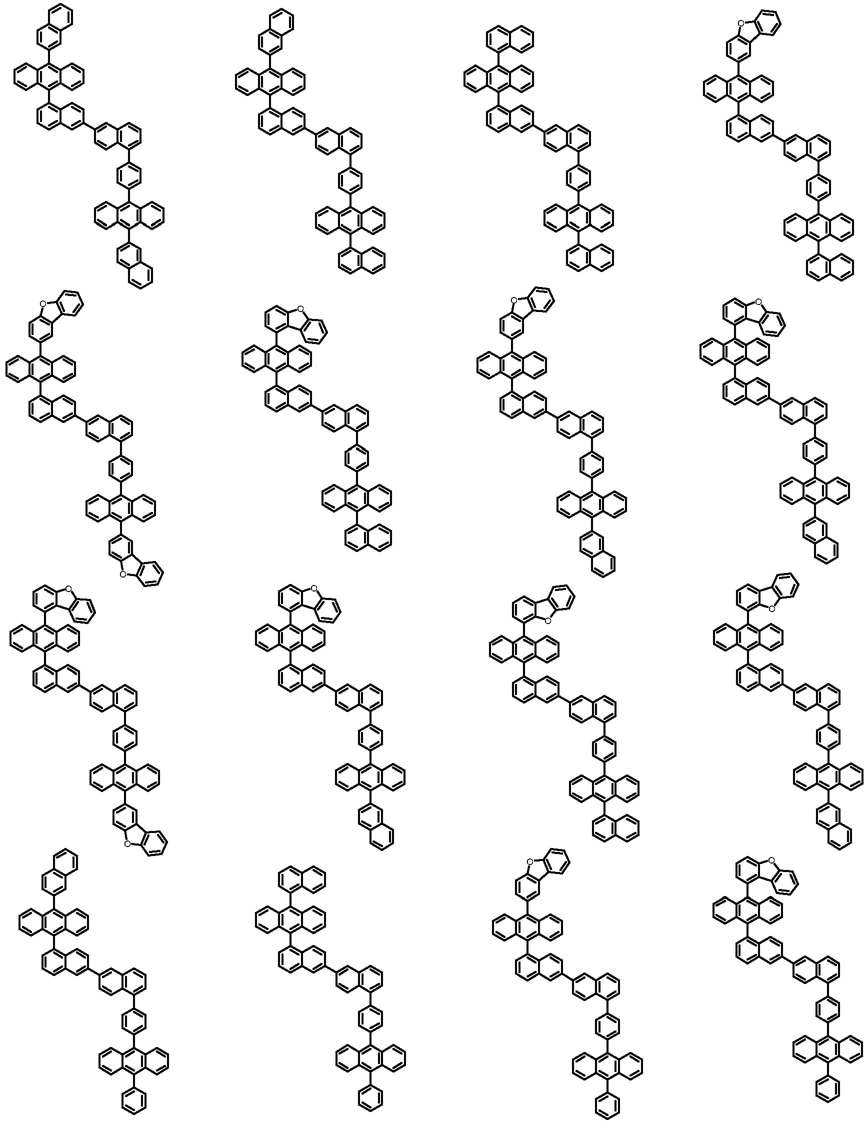

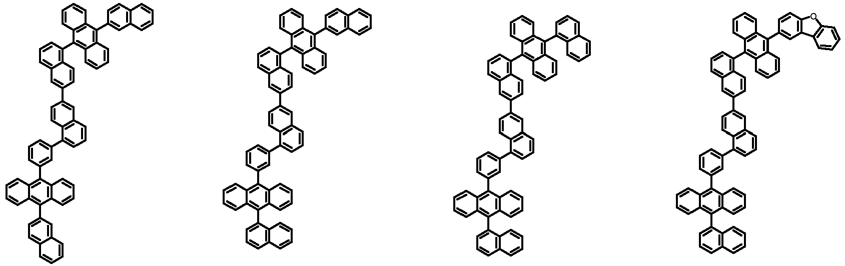

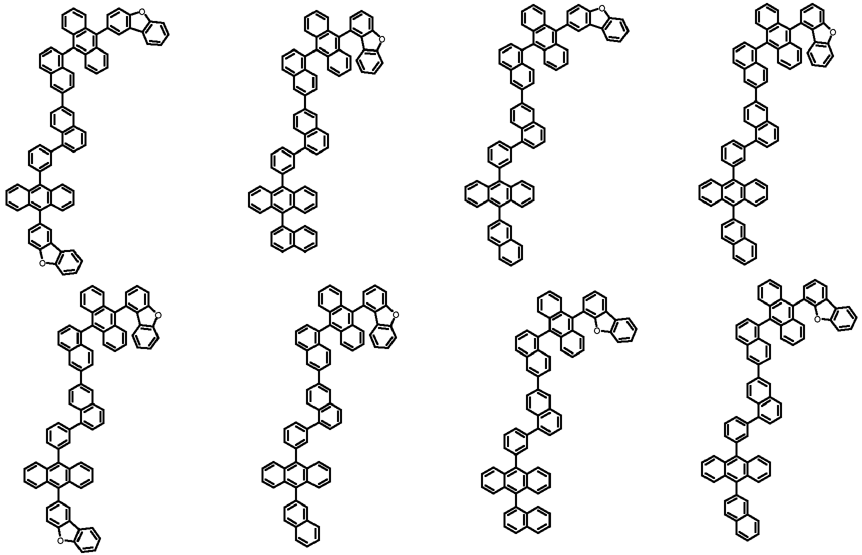

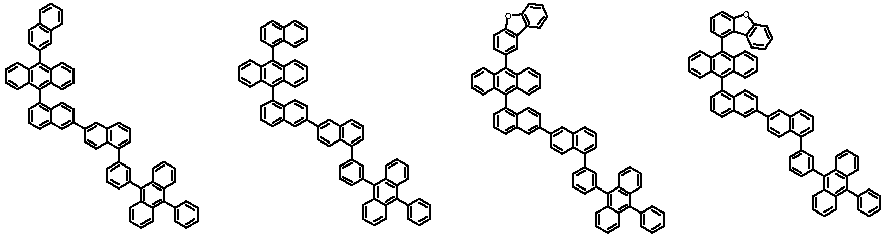

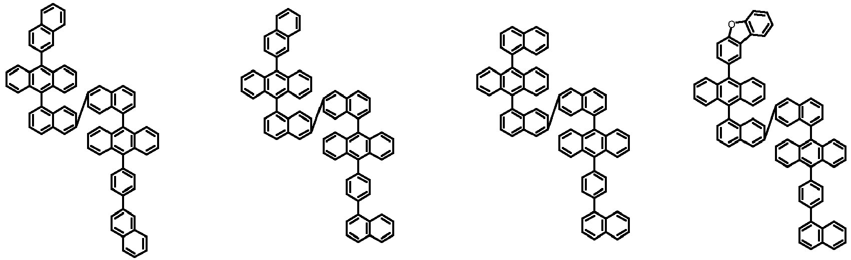

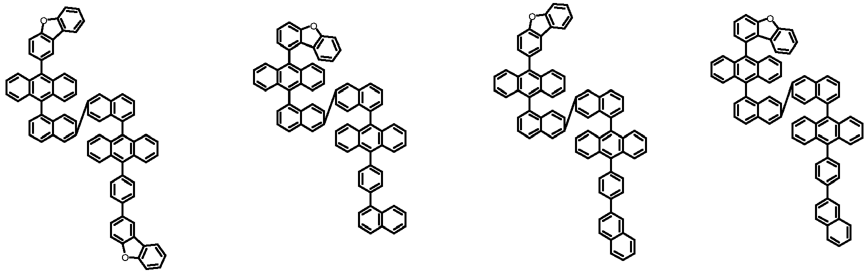

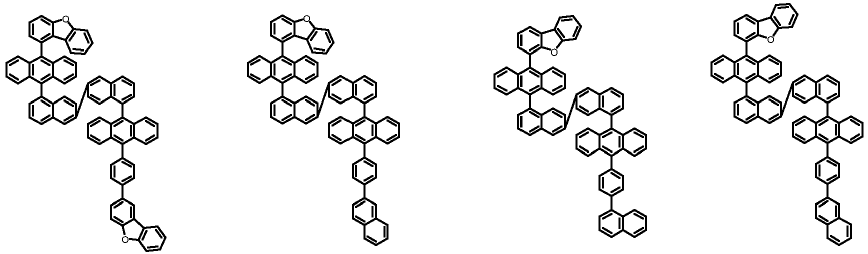

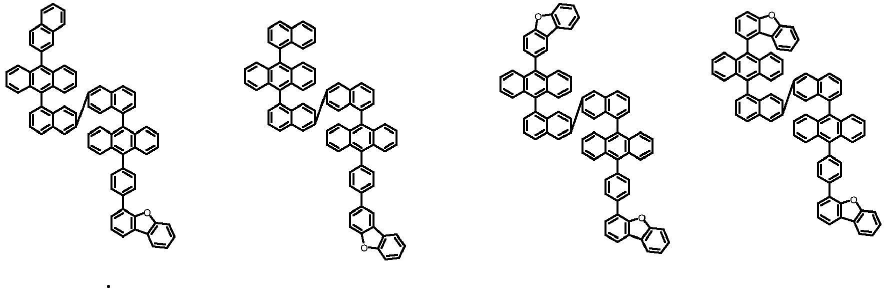

On the other hand, the compound represented by Chemical Formula 1, where a, b, c, and d are 0, can be prepared by a preparation method as shown in the following Reaction Scheme 1 as an example. 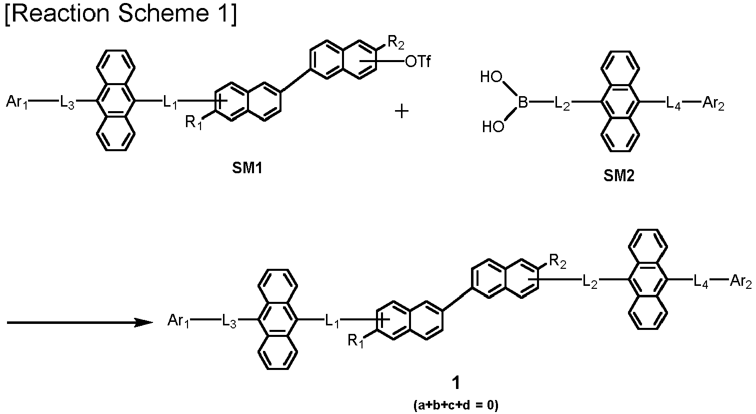 in Reaction Scheme 1, the definitions of each substituent are the same as described above.

Specifically, the compound represented by Chemical Formula 1 can be prepared through a Suzuki coupling reaction between Compound SM1 having an introduced -OTf (-O₃SCF₃) group which is a reactive group for the Suzuki coupling reaction and Compound SM2. At this time, the Suzuki coupling reaction is preferably carried out in the presence of a palladium catalyst and a base. Further, a reactive group for the Suzuki coupling reaction can be changed as appropriate.

In addition, the compound represented by Chemical Formula 1 having at least one deuterium (namely, a compound in which n is an integer greater than 0 (n is an integer of 1 or more) in Chemical Formula 1D) can be prepared by a preparation method shown in the following Reaction Scheme 2. 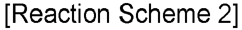 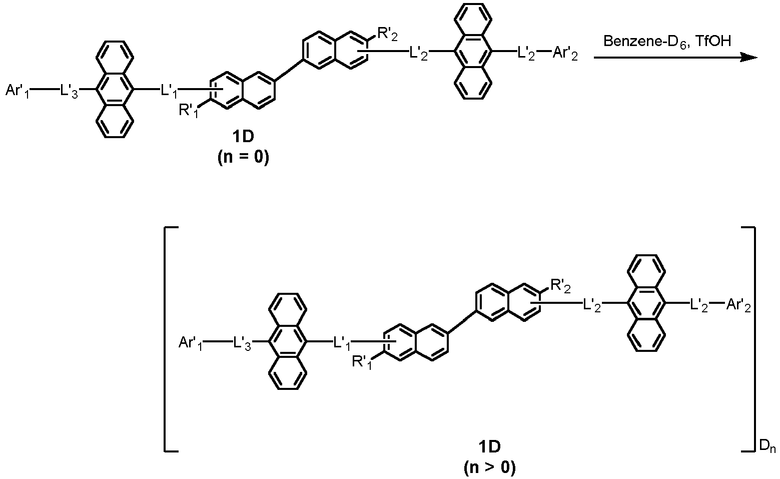 in Reaction Scheme 2, the definitions of each substituent are the same as described above.

Specifically, the compound represented by Chemical Formula 1 having at least one deuterium can be prepared by deuterating a compound represented by Chemical Formula 1 that is not substituted with deuterium. At this time, the deuterium substitution reaction can be performed by introducing a compound represented by Chemical Formula 1 that is not substituted with deuterium into a deuterated solvent such as a benzene-D₆ (C₆D₆) solution, and then reacting it with trifluoromethanesulfonic acid (TfOH).

Meanwhile, the compound represented by Chemical Formula 1 can be prepared by appropriately changing the reactants in Reaction Schemes 1 and 2, and the preparation method thereof can be further embodied in Preparation Examples described later.

Meanwhile, the compound represented by Chemical Formula 1 has high solubility in an organic solvent used in the solution process, for example, an organic solvent such as cyclohexanone. Thus, it is suitable for use in a large-scale solution process such as an inkjet coating method using a solvent having a high boiling point.

The organic material layer containing the compound according to the present disclosure can be formed by using various methods such as vacuum deposition, solution process, and the like, and the solution process will be described in detail below.

### (Coating Composition)

On the other hand, the compound according to the present disclosure can form an organic material layer, particularly a light emitting layer, of an organic light emitting device by a solution process. Specifically, the compound may be used as a host material of the light emitting layer. For this purpose, the present disclosure provides a coating composition including the compound according to the present disclosure and a solvent.

The solvent is not particularly limited as long as it is a solvent capable of dissolving or dispersing the compound according to the present disclosure. Examples of the solvent may include chlorine-based solvents such as chloroform, methylene chloride, 1,2-dichloroethane, 1,1,2-trichloroethane, chlorobenzene and o-dichlorobenzene; ether-based solvents such as tetrahydrofuran and dioxane; aromatic hydrocarbon-based solvents such as toluene, xylene, trimethylbenzene, mesitylene, 1-methylnaphthalene and 2-methylnaphthalene; aliphatic hydrocarbon-based solvents such as cyclohexane, methylcyclohexane, n-pentane, n-hexane, n-heptane, n-octane, n-nonane and n-decane; ketone-based solvents such as acetone, methyl ethyl ketone, and cyclohexanone; ester-based solvents such as ethyl acetate, butyl acetate and ethyl cellosolve acetate; polyalcohols such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin and 1,2-hexanediol, and derivatives thereof; alcohol-based solvents such as methanol, ethanol, propanol, isopropanol and cyclohexanol; sulfoxide-based solvents such as dimethyl sulfoxide; amide-based solvents such as N-methyl-2-pyrrolidone and N,N-dimethylformamide; benzoate-based solvents such as butylbenzoate, methyl-2-methoxybenzoate, and ethyl benzoate; phthalate-based solvents such as dimethyl phthalate, diethyl phthalate, and diphenyl phthalate; tetraline; solvent such as 3-phenoxytoluene, and the like. In addition, the above-mentioned solvents may be used singly or in combination of two or more solvents. Preferably, cyclohexanone may be used as the solvent.

Further, the coating composition may further include a compound used as a dopant material, and the details of the compound used for the dopant material will be described later.

Further, the viscosity of the coating composition is preferably 1 cP or more. Also, in consideration of the easiness of coating the coating composition, the viscosity of the coating composition is preferably 10 cP or less. Further, the concentration of the compound according to the present disclosure in the coating composition is preferably 0.1 wt/v% or more. Further, the concentration of the compound according to the present disclosure in the coating composition is preferably 20 wt/v% or less, so that the coating composition can optimally coated.

Further, the solubility (wt%) of the compound represented by Chemical Formula 1 at room temperature/atmospheric pressure may be 0.1 wt% or more, more specifically 0.1 wt% to 5 wt%, based on the solvent cyclohexanone. Thereby, the coating composition containing the compound represented by Chemical Formula 1 and the solvent can be used in a solution process.

According to the present disclosure, there is provided a method for forming a light emitting layer using the above-mentioned coating composition. Specifically, the method includes the steps of: coating the above-mentioned light emitting layer according to the present disclosure onto the anode, or onto a hole transport layer formed on the anode by a solution process; and heat-treating the coated coating composition.

The solution process uses the above-mentioned coating composition according to the present disclosure, and refers to spin coating, dip coating, doctor blading, inkjet printing, screen printing, spray method, roll coating, and the like, but is not limited thereto.

The heat treatment temperature in the heat treatment step is preferably from 150 to 230°C. Further, a heat treatment time may be 1 minute to 3 hours, more preferably 10 minutes to 1 hour. Further, the heat treatment is preferably carried out in an inert gas atmosphere such as argon and nitrogen.

### (Organic Light Emitting Device)

On the other hand, according to the present disclosure, there is provided an organic light emitting device comprising a compound represented by Chemical Formula 1. In one example, the present disclosure provides an organic light emitting device comprising: a first electrode; a second electrode that is provided opposite to the first electrode; and one or more organic material layers that are provided between the first electrode and the second electrode, wherein one or more layers of the organic material layers includes the compound represented by Chemical Formula 1.

The organic material layer of the organic light emitting device of the present disclosure may have a single-layer structure, or it may have a multilayered structure in which two or more organic material layers are stacked. For example, the organic light emitting device of the present disclosure may have a structure comprising a hole injection layer, a hole transport layer, a light emitting layer, an electron transport layer, an electron injection layer and the like as the organic material layer. However, the structure of the organic light emitting device is not limited thereto, and it may include a smaller number of organic layers.

In one embodiment, the organic material layer may include a light emitting layer, wherein the organic material layer including the compound may be a light emitting layer.

In another embodiment, the organic material layer may include a hole injection layer, a hole transport layer, a light emitting layer, and an electron injection and transport layer, wherein the organic material layer including the compound may be a light emitting layer or an electron injection and transport layer.

In yet another embodiment, the organic material layer may include a hole injection layer, a hole transport layer, an electron blocking layer, a light emitting layer, and an electron injection and transport layer, wherein the organic material layer including the compound may be a light emitting layer or an electron injection and transport layer.

In yet another embodiment, the organic material layer may include a hole injection layer, a hole transport layer, an electron blocking layer, a light emitting layer, an electron inhibition layer, and an electron injection and transport layer, wherein the organic material layer including the compound may be a light emitting layer or an electron injection and transport layer.

The organic light emitting device according to the present disclosure may have a single-layer structure, or it may have a multilayered structure in which two or more organic material layers are stacked. For example, the organic light emitting device of the present disclosure may have a structure that comprises a hole injection layer and a hole transport layer between the first electrode and the light emitting layer, and an electron transport layer and an electron injection layer between the light emitting layer and the second electrode, in addition to the light emitting layer as the organic material layer. However, the structure of the organic light emitting device is not limited thereto, and it may include a smaller number of organic layers or a larger number of organic layers.

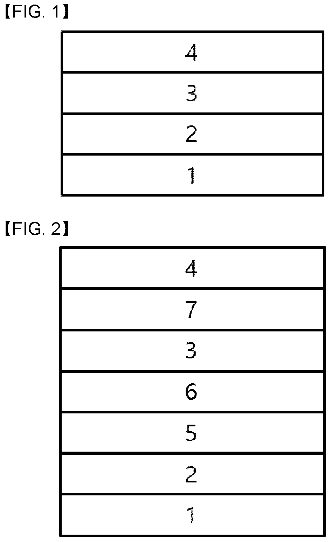

Further, the organic light emitting device according to the present disclosure may be a normal type organic light emitting device in which an anode, one or more organic material layers and a cathode are sequentially stacked on a substrate in which the first electrode is an anode and the second electrode is a cathode. Further, the organic light emitting device according to the present disclosure may be an inverted type organic light emitting device in which a cathode, one or more organic material layers and an anode are sequentially stacked on a substrate in which the first electrode is a cathode and the second electrode is an anode. For example, the structure of an organic light emitting device according to an embodiment of the present disclosure is illustrated in FIGS. 1 and 2.

FIG. 1 shows an example of an organic light emitting device comprising a substrate 1, an anode 2, a light emitting layer 3, and a cathode 4. In such a structure, the compound represented by Chemical Formula 1 may be included in the light emitting layer.

FIG. 2 shows an example of an organic light emitting device comprising a substrate 1, an anode 2, a hole injection layer 5, a hole transport layer 6, a light emitting layer 3, an electron injection and transport layer 7, and a cathode 4. In such a structure, the compound represented by Chemical Formula 1 may be included in the light emitting layer.

The organic light emitting device according to the present disclosure may be manufactured by materials and methods known in the art, except that the light emitting layer includes the compound according to the present disclosure and is manufactured as described above.

For example, the organic light emitting device according to the present disclosure can be manufactured by sequentially stacking an anode, an organic material layer and a cathode on a substrate. In this case, the organic light emitting device may be manufactured by depositing a metal, metal oxides having conductivity, or an alloy thereof on the substrate using a PVD (physical vapor deposition) method such as a sputtering method or an e-beam evaporation method to form an anode, forming organic material layers including the hole injection layer, the hole transport layer, the light emitting layer and the electron transport layer thereon, and then depositing a material that can be used as the cathode thereon.

In addition to such a method, the organic light emitting device may be manufactured by sequentially depositing a cathode material, an organic material layer and an anode material on a substrate (International Publication WO2003/012890). However, the manufacturing method is not limited thereto.

In one example, the first electrode is an anode, and the second electrode is a cathode, or alternatively, the first electrode is a cathode, and the second electrode is an anode.

As the anode material, generally, a material having a large work function is preferably used so that holes can be smoothly injected into the organic material layer. Specific examples of the anode material include metals such as vanadium, chrome, copper, zinc, and gold, or an alloy thereof; metal oxides such as zinc oxides, indium oxides, indium tin oxides (ITO), and indium zinc oxides (IZO); a combination of metals and oxides, such as ZnO:Al or SnO₂:Sb; conductive polymers such as poly(3-methylthiophene), poly[3,4-(ethylene-1,2-dioxy)thiophene](PEDOT), polypyrrole, and polyaniline, and the like, but are not limited thereto.

As the cathode material, generally, a material having a small work function is preferably used so that electrons can be easily injected into the organic material layer. Specific examples of the cathode material include metals such as magnesium, calcium, sodium, potassium, titanium, indium, yttrium, lithium, gadolinium, aluminum, silver, tin, and lead, or an alloy thereof; a multilayered structure material such as LiF/Al or LiO₂/Al, and the like, but are not limited thereto.

The hole injection layer is a layer for injecting holes from the electrode, and the hole injection material is preferably a compound which has a capability of transporting the holes, thus has a hole injecting effect in the anode and an excellent hole injecting effect to the light emitting layer or the light emitting material, prevents excitons produced in the light emitting layer from moving to a hole injection layer or the electron injection material, and further is excellent in the ability to form a thin film. It is preferable that a HOMO (highest occupied molecular orbital) of the hole injection material is between the work function of the anode material and a HOMO of a peripheral organic material layer. Specific examples of the hole injection material include metal porphyrin, oligothiophene, an arylamine-based organic material, a hexanitrilehexaazatriphenylene-based organic material, a quinacridone-based organic material, a perylene-based organic material, anthraquinone, polyaniline and polythiophene-based conductive compound, and the like, but are not limited thereto.

The hole transport layer is a layer that receives holes from a hole injection layer and transports the holes to the light emitting layer. The hole transport layer is suitably a material having large mobility to the holes, which may receive holes from the anode or the hole injection layer and transfer the holes to the light emitting layer. As the hole transport material, the compound represented by Chemical Formula 1 may be used, or an arylamine-based organic material, a conductive polymer, a block copolymer in which a conjugate portion and a non-conjugate portion are present together, and the like may be used, but are not limited thereto.

Meanwhile, the organic light emitting device may be provided with an electron inhibition layer between the hole transport layer and the light emitting layer. The electron inhibition layer refers to a layer which is formed on the hole transport layer, and preferably, is provided in contact with the light emitting layer, and thus serves to control hole mobility, to prevent excessive movement of electrons, and to increase the probability of hole-electron bonding, thereby improving the efficiency of the organic light emitting device. The electron inhibition layer includes an electron blocking material, and as an example of such an electron blocking material, a compound represented by the Chemical Formula 1 may be used, or an arylamine-based organic material and the like may be used, but is not limited thereto.

The light emitting layer may include a host material and a dopant material. As the host material, the above-mentioned compound represented by Chemical Formula 1 can be used. Further, the host material may be a fused aromatic ring derivative, a heterocycle-containing compound or the like together with the compound represented by Chemical Formula 1. Specific examples of the fused aromatic ring derivatives include anthracene derivatives, pyrene derivatives, naphthalene derivatives, pentacene derivatives, phenanthrene compounds, fluoranthene compounds, and the like. Examples of the heterocyclic-containing compounds include carbazole derivatives, dibenzofuran derivatives, ladder-type furan compounds, pyrimidine derivatives, and the like, but are not limited thereto.

Further, examples of the dopant material include an aromatic amine derivative, a styrylamine compound, a boron complex, a fluoranthene compound, a metal complex, and the like. Specifically, the aromatic amine derivative is a substituted or unsubstituted fused aromatic ring derivative having an arylamino group, and examples thereof include pyrene, anthracene, chrysene, periflanthene and the like, which have an arylamino group. The styrylamine compound is a compound where at least one arylvinyl group is substituted in substituted or unsubstituted arylamine, in which one or two or more substituent groups selected from the group consisting of an aryl group, a silyl group, an alkyl group, a cycloalkyl group, and an arylamino group are substituted or unsubstituted. Specific examples thereof include styrylamine, styryldiamine, styryltriamine, styryltetramine, and the like, but are not limited thereto. Further, the metal complex includes an iridium complex, a platinum complex, and the like, but is not limited thereto.

Meanwhile, the organic light emitting device may be provided with a hole blocking layer between the light emitting layer and the electron transport layer. The hole blocking layer refers to a layer which is formed on the light emitting layer, and preferably, is provided in contact with the light emitting layer, and thus severs to control electron mobility, to prevent excessive movement of holes, and to increase the probability of hole-electron bonding, thereby improving the efficiency of the organic light emitting device. The hole blocking layer includes a hole blocking material, and as an example of such hole blocking material, a compound into which an electron-withdrawing group is introduced, such as azine derivatives including triazine; triazole derivatives; oxadiazole derivatives; phenanthroline derivatives; phosphine oxide derivatives can be used, but is not limited thereto.

The electron injection and transport layer is a layer for simultaneously performing the roles of an electron transport layer and an electron injection layer that inject electrons from an electrode and transport the received electrons up to the light emitting layer, and is formed on the light emitting layer or the hole blocking layer. The electron injection and transport material is suitably a material which can receive electrons well from a cathode and transfer the electrons to a light emitting layer, and has a large mobility for electrons. Specific examples of the electron injection and transport material include: an Al complex of 8-hydroxyquinoline; a complex including Alq₃; an organic radical compound; a hydroxyflavone-metal complex, a triazine derivative, and the like, but are not limited thereto. Alternatively, it may be used together with fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, a metal complex compound, a nitrogen-containing 5-membered ring derivative, and the like, but are not limited thereto.

The electron injection and transport layer may also be formed as a separate layer such as an electron injection layer and an electron transport layer. In such a case, the electron transport layer is formed on the light emitting layer or the hole blocking layer, and the above-mentioned electron injection and transport material may be used as the electron transport material included in the electron transport layer. In addition, the electron injection layer is formed on the electron transport layer, and examples of the electron injection material included in the electron injection layer include LiF, NaCl, CsF, Li₂O, BaO, fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, a metal complex compound, a nitrogen-containing 5-membered ring derivative, and the like.

Examples of the metal complex compound include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium, and the like, but are not limited thereto.

In addition to the materials as mentioned above, the light emitting layer, the hole injection layer, the hole transport layer, the electron transport layer, and the electron injection layer may further include an inorganic compound or a polymer compound such as quantum dots.

The quantum dots may be, for example, colloidal quantum dots, alloy quantum dots, core-shell quantum dots, or core quantum dots. It may be a quantum dot including elements belonging to groups 2 and 16, elements belonging to groups 13 and 15, elements belonging to groups 13 and 17, elements belonging to groups 11 and 17, or elements belonging to groups 14 and 15. Quantum dots including elements such as cadmium (Cd), selenium (Se), zinc (Zn), sulfur (S), phosphorus (P), indium (In), tellurium (Te), lead (Pb), gallium (Ga), arsenic (As) may be used.

The organic light emitting device according to the present disclosure may be a bottom emission device, a top emission device, or a double-sided light emitting device, and in particular, may be a bottom emission device that requires relatively high luminous efficiency.

In addition, the compound according to the present disclosure may be included in an organic solar cell or an organic transistor in addition to an organic light emitting device.

The preparation of the compound represented by Chemical Formula 1 and the organic light emitting device including the same will be specifically described in the following Examples. However, the following Examples are provided for illustrative purposes only, and are not intended to limit the scope of the present disclosure.

### Preparation Example 1: Preparation of Compound 1

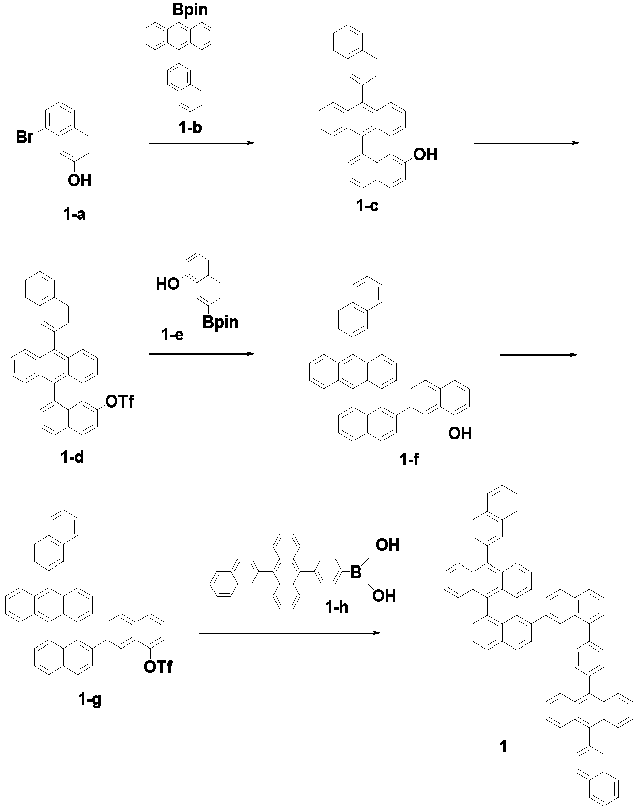

Compound 1-a (1.0 eq.) and Compound 1-b (1.1 eq.) were placed in a round bottom flask, and dissolved in THF. Cs₂CO₃ (5.0 eq.) dissolved in water was injected. Pd(PPh₃)₄ (10 mol%) was added dropwise thereto under a bath temperature of 80°C, and stirred for 3 days. After the reaction, the reaction mixture was cooled at room temperature, sufficiently diluted with EtOAc, and washed with EtOAc/brine, and the organic layer was separated. Water was removed with MgSO₄ and passed through a Celite-Florisil-Silica pad. The passed solution was concentrated under reduced pressure, and then purified by column chromatography to obtain Compound 1-c (Yield: 73%).

Compound 1-c (1.0 eq.) was placed in a round bottom flask, and dissolved in anhydrous CH₂Cl₂. Then, pyridine (2.0 eq.) was added dropwise thereto at room temperature, the bath temperature was lowered to 0°C, and the mixture was stirred for 10 minutes. Tf₂O (1.2 eq.) dissolved in anhydrous CH₂Cl₂ was slowly added dropwise to the mixture using a dropping funnel, the bath temperature was gradually raised from 0°C to room temperature, and then the mixture was stirred overnight. After the reaction, the reaction mixture was sufficiently diluted with CH₂Cl₂, and then washed with CH₂Cl₂/brine, and the organic layer was separated. Water was removed with MgSO₄ and passed through a Celite-Florisil-Silica pad. The passed solution was concentrated under reduced pressure, and then purified by column chromatography to prepare Compound 1-d (Yield: 99%).

Compound 1-d (1.0 eq.) and Compound 1-e (1.1 eq.) were placed in a round bottom flask, and dissolved in THF. Na₂CO₃ (3.0 eq.) dissolved in water was injected. Pd(PPh₃)₄ (10 mol%) was added dropwise thereto under a bath temperature of 80°C, and the mixture was stirred overnight. After the reaction, the reaction mixture was cooled at room temperature, sufficiently diluted with EtOAc, and then washed with EtOAc/brine. The organic layer was separated, water was removed with MgSO₄ and passed through a Celite-Florisil-Silica pad. The passed solution was concentrated under reduced pressure, and then purified by column chromatography to prepare Compound 1-f (Yield: 71%).

Compound 1-f (1.0 eq.) was placed in a round bottom flask and dissolved in anhydrous CH₂Cl₂. Then, pyridine (2.0 eq.) was added dropwise thereto at room temperature, the bath temperature was lowered to 0°C, and the mixture was stirred for 10 minutes. Then, Tf₂O (1.2 eq.) dissolved in anhydrous CH₂Cl₂ was slowly added dropwise to the mixture using a dropping funnel, the bath temperature was gradually raised from 0°C to room temperature, and then the mixture was stirred overnight. After the reaction, the reaction mixture was sufficiently diluted with CH₂Cl₂, and then washed with CH₂Cl₂/brine, and the organic layer was separated. Water was removed with MgSO₄ and passed through a Celite-Florisil-Silica pad. The passed solution was concentrated under reduced pressure, and then purified by column chromatography to prepare Compound 1-g (Yield: 99%).

Compound 1-g (1.0 eq.) and Compound 1-h (1.1 eq.) were placed in a round bottom flask, and dissolved in THF. Na₂CO₃ (3.0 eq.) dissolved in water was injected. Pd(PPh₃)₄ (10 mol%) was added dropwise thereto under a bath temperature of 80°C, and stirred overnight. After the reaction, the reaction mixture was cooled at room temperature, sufficiently diluted with EtOAc, and washed with EtOAc/brine, and the organic layer was separated. Water was removed with MgSO₄ and passed through a Celite-Florisil-Silica pad. The passed solution was concentrated under reduced pressure, and then purified by column chromatography to obtain Compound 1 (Yield: 84%).
m/z [M+H]⁺ 935.4

### Preparation Example 2: Preparation of Compound 2

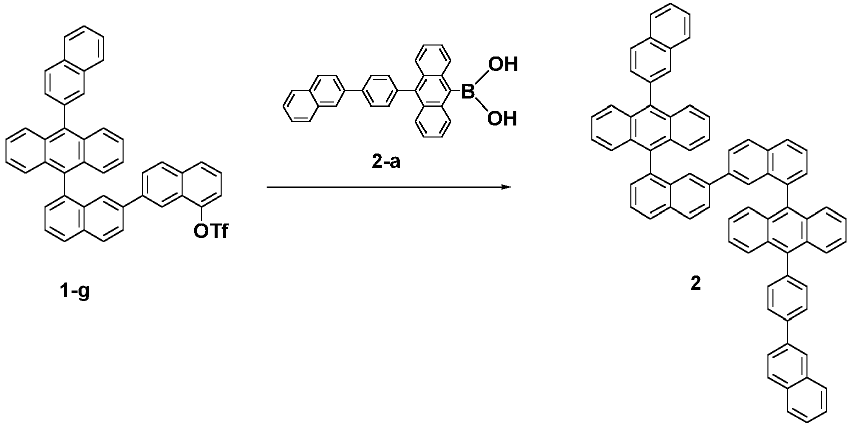

Compound 2 was prepared in the same manner as in Compound 1, except that in the synthesis, Compound 2-a was used instead of Compound 1-h.
m/z [M+H]⁺ 935.4

### Preparation Example 3: Preparation of Compound 3

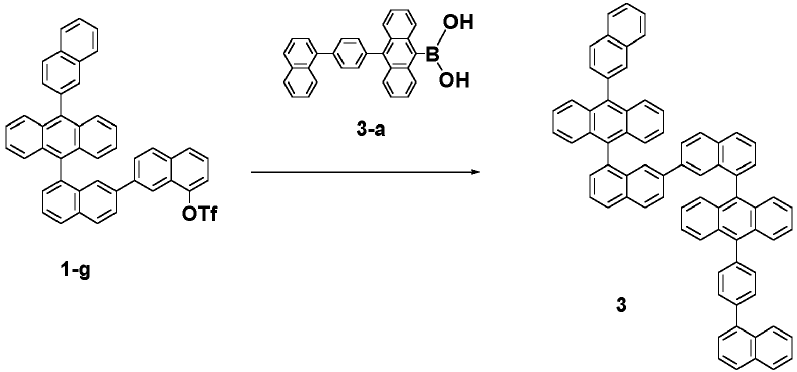

Compound 3 was prepared in the same manner as in Compound 1, except that in the synthesis, Compound 3-a was used instead of Compound 1-h.
m/z [M+H]⁺ 935.4

### Preparation Example 4: Preparation of Compound 4

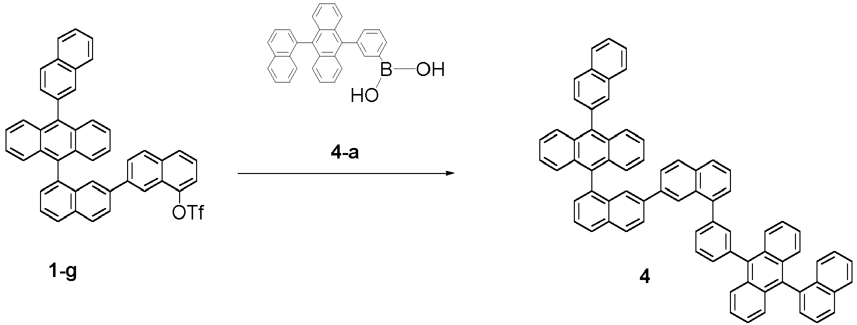

Compound 4 was prepared in the same manner as in Compound 1, except that in the synthesis, Compound 4-a was used instead of Compound 1-h.
m/z [M+H]⁺ 935.4

### Preparation Example 5: Preparation of Compound 5

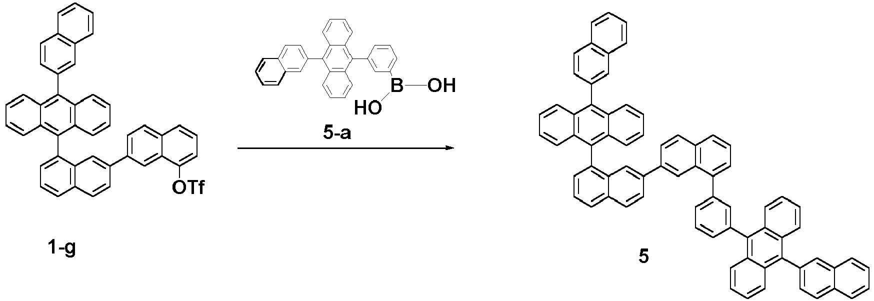

Compound 5 was prepared in the same manner as in Compound 1, except that in the synthesis, Compound 5-a was used instead of Compound 1-h.
m/z [M+H]⁺ 935.4

### Preparation Example 6: Preparation of Compound 6

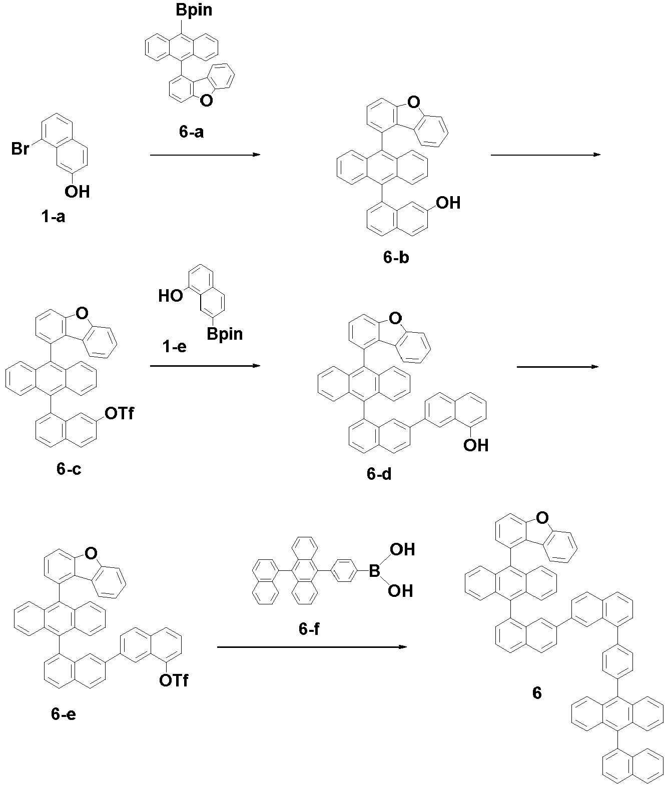

Compound 6 was prepared in the same manner as in Compound 1, except that in the synthesis, Compound 6-a was used instead of Compound 1-b, Compound 6-b instead of Compound 1-c, Compound 6-c instead of Compound 1-d, Compound 6-d instead of Compound 1-f, Compound 6-e instead of Compound 1-g, and Compound 6-f instead of Compound 1-h.
m/z [M+H]⁺ 975.4

### Preparation Example 7: Preparation of Compound 7

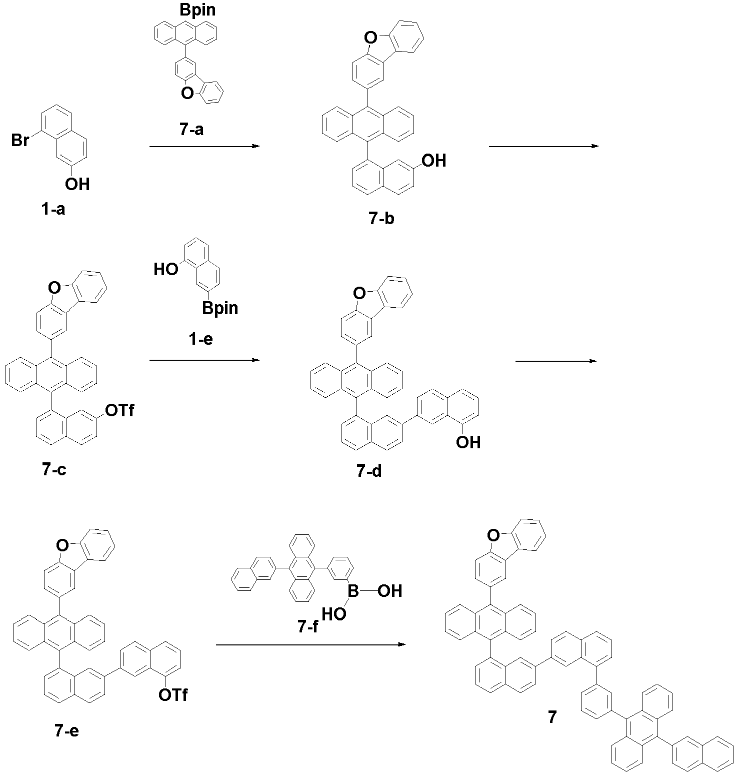

Compound 7 was prepared in the same manner as in Compound 1, except that in the synthesis, Compound 7-a was used instead of Compound 1-b, Compound 7-b instead of Compound 1-c, Compound 7-c instead of Compound 1-d, Compound 7-d instead of Compound 1-f, Compound 7-e instead of Compound 1-g, and Compound 7-f instead of Compound 1-h.
m/z [M+H]⁺ 975.4

### Preparation Example 8: Preparation of Compound 8

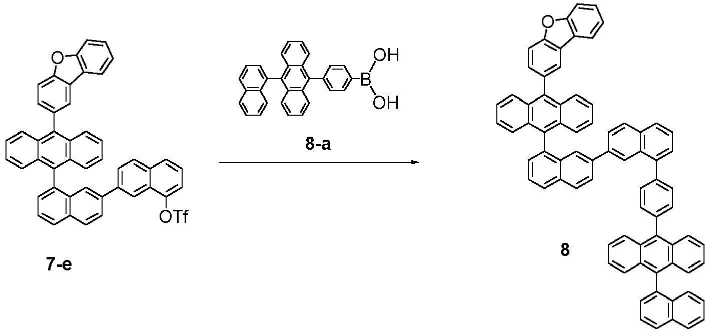

Compound 8 was prepared in the same manner as in Compound 7, except that in the synthesis, Compound 8-a was used instead of Compound 7-f.
m/z [M+H]⁺ 975.4

### Preparation Example 9: Preparation of Compound 9

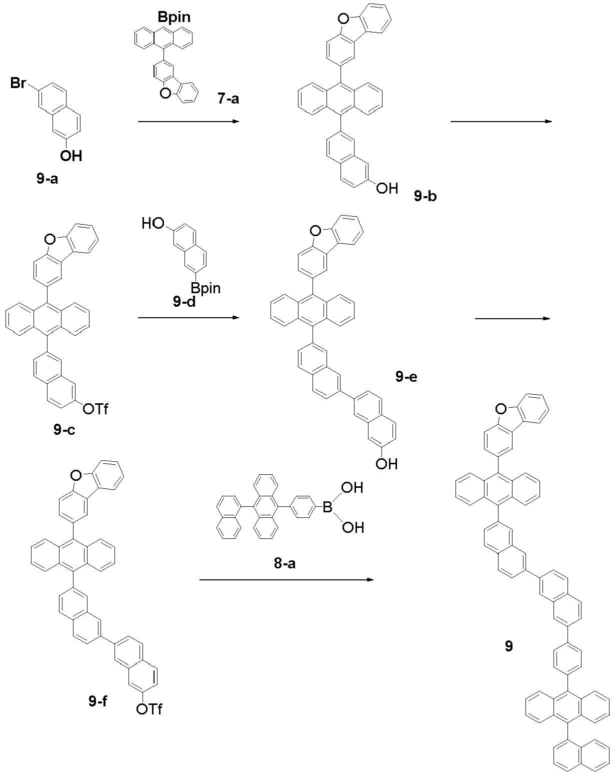

Compound 9 was prepared in the same manner as in Compound 1, except that in the synthesis, Compound 9-a was used instead of Compound 1-a, Compound 7-a instead of Compound 1-b, Compound 9-b instead of Compound 1-c, Compound 9-c instead of Compound 1-d, Compound 9-d instead of Compound 1-e, Compound 9-e instead of Compound 1-f, Compound 9-f instead of Compound 1-g, and Compound 8-a instead of Compound 1-h.
m/z [M+H]⁺ 975.4

### Preparation Example 10: Preparation of Compound 10

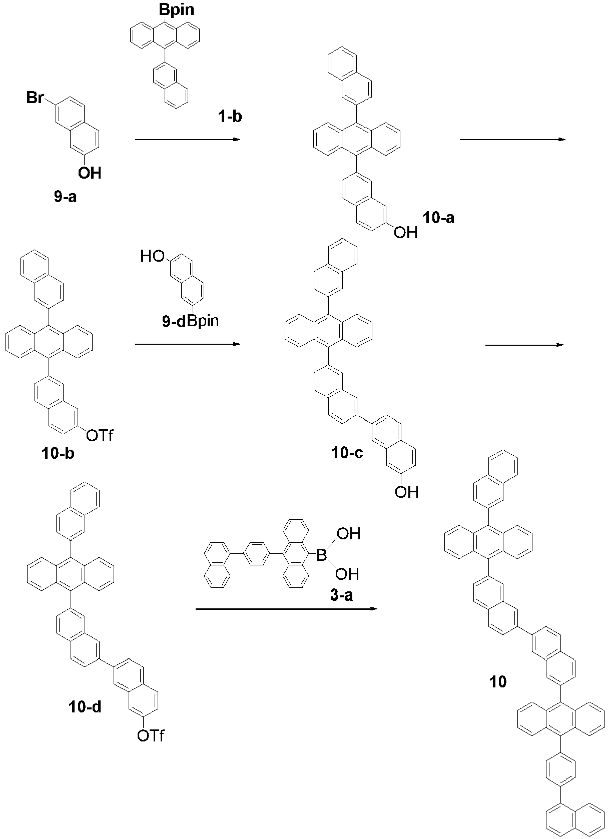

Compound 10 was prepared in the same manner as in Compound 1, except that in the synthesis, Compound 9-a was used instead of Compound 1-a, Compound 10-a instead of Compound 1-c, Compound 10-b instead of Compound 1-d, Compound 9-d instead of Compound 1-e, Compound 10-c instead of Compound 1-f, Compound 10-d instead of Compound 1-g, and Compound 3-a instead of Compound 1-h.
m/z [M+H]⁺ 935.4

### Preparation Example 11: Preparation of Compound 11

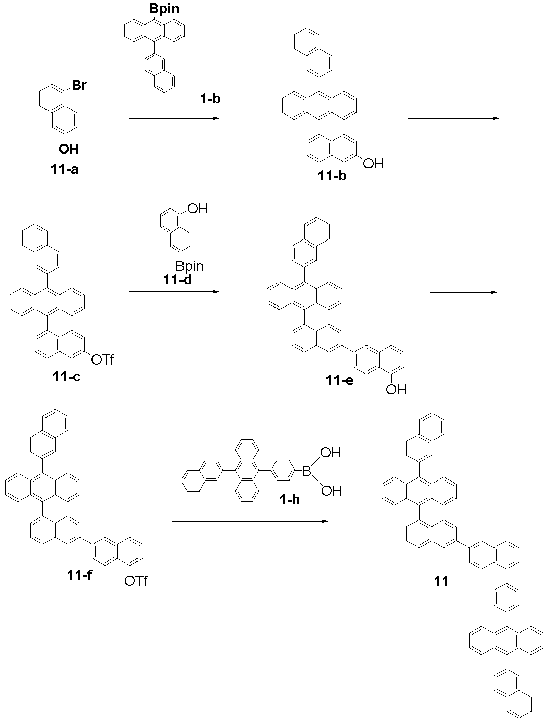

Compound 11 was prepared in the same manner as in Compound 1, except that in the synthesis, Compound 11-a was used instead of Compound 1-a, Compound 11-b instead of Compound 1-c, Compound 11-c instead of Compound 1-d, Compound 11-d instead of Compound 1-e, Compound 11-e instead of Compound 1-f, and Compound 11-f instead of Compound 1-g.
m/z [M+H]⁺ 935.4

### Preparation Example 12: Preparation of Compound 12

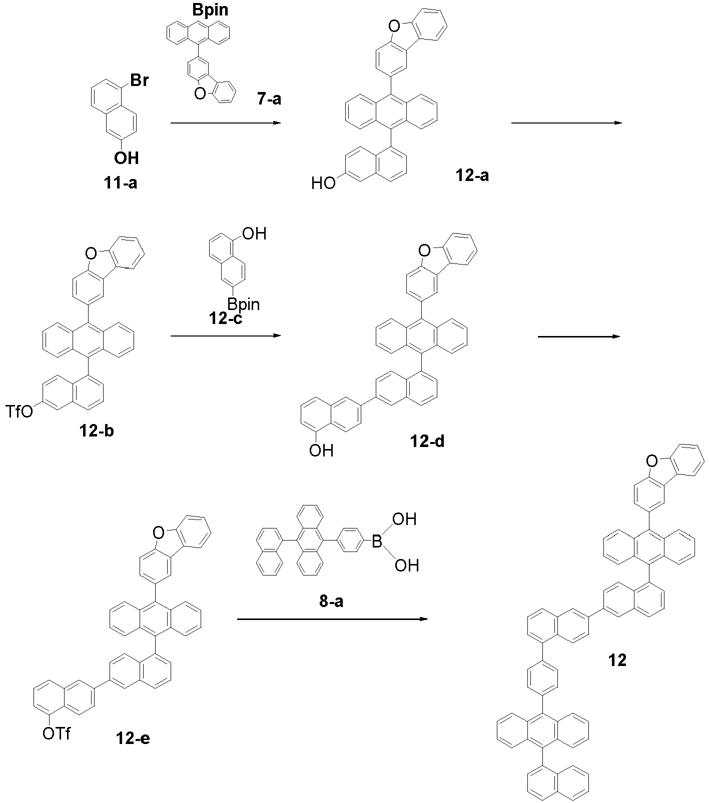

Compound 12 was prepared in the same manner as in Compound 1, except that in the synthesis, Compound 11-a was used instead of Compound 1-a, Compound 7-a instead of Compound 1-b, Compound 12-a instead of Compound 1-c, Compound 12-b instead of Compound 1-d, Compound 12-c instead of Compound 1-e, Compound 12-d instead of Compound 1-f, Compound 12-e instead of Compound 1-g, and Compound 8-a instead of Compound 1-h.
m/z [M+H]⁺ 975.4

### Preparation Example 13: Preparation of Compound 13

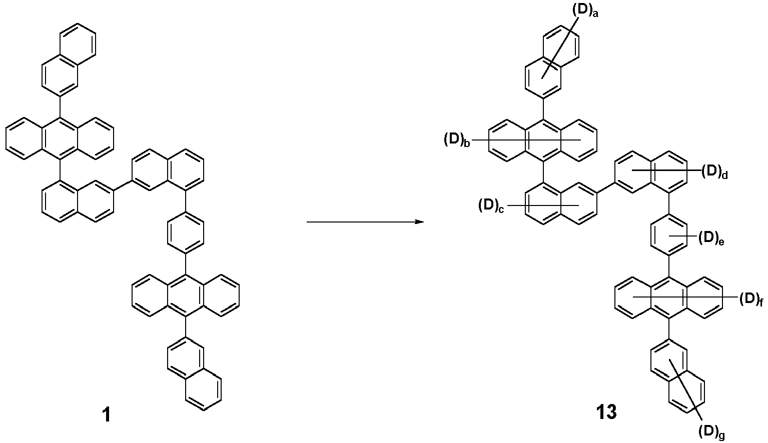

Compound 1 (1.0 eq.) was placed in a round bottom flask under a nitrogen atmosphere, and dissolved in benzene-D₆ (150 eq.). Trifluoromethanesulfonic acid (TfOH, 2.0 eq.) was slowly added dropwise to the reaction solution, and the mixture was stirred at 25°C for 2 hours. D₂O was dropwise added to the reaction solution to terminate the reaction, and potassium phosphate tribasic (30 wt% in aqueous solution, 2.4 eq.) was added dropwise thereto, and the pH of the aqueous layer was adjusted to 9-10. The reaction mixture was washed with CH₂Cl₂/DI water, and the organic layer was separated. Water was removed with MgSO₄ and passed through a Celite-Florisil-Silica pad. The passed solution was concentrated under reduced pressure and purified by column chromatography to prepare Compound 13 (a+b+c+d+e+f+g=40-46, yield: 87%, deuterium substitution rate: 87.0-100%). At this time, the deuterium substitution rate was calculated as the number of substituted deuterium relative to the total number of hydrogens that can be present in the compound, after the number of substituted deuterium in the compound was obtained through MALDI-TOF MS (Matrix-Assisted Laser Desorption/Ionization Time-of-Flight Mass Spectrometer) analysis.

### Preparation Example 14: Preparation of Compound 14

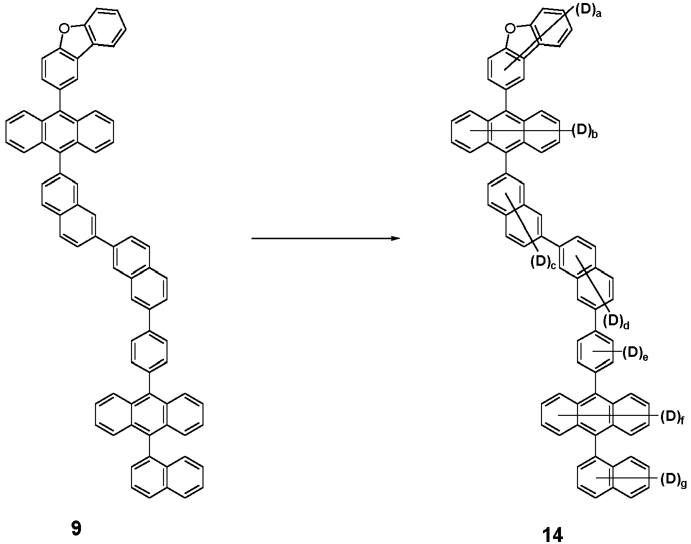

Compound 9 (1.0 eq.) was placed in a round bottom flask under a nitrogen atmosphere, and dissolved in benzene-D₆ (150 eq.). TfOH (2.0 eq.) was slowly added dropwise to the reaction solution, and the mixture was stirred at 25°C for 2 hours. D₂O was dropwise added to the reaction solution to terminate the reaction, and potassium phosphate tribasic (30 wt% in aqueous solution, 2.4 eq.) was added dropwise thereto, and the pH of the aqueous layer was adjusted to 9-10. The reaction mixture was washed with CH₂Cl₂/DI water and the organic layer was separated. Water was removed with MgSO₄ and passed through a Celite-Florisil-Silica pad. The passed solution was concentrated under reduced pressure and purified by column chromatography to prepare Compound 14 (a+b+c+d+e+f+g=40-46, yield: 85%, deuterium substitution rate: 87.0-100%). At this time, the deuterium substitution rate was calculated in the same manner as in Compound 12.

### Comparative Preparation Example 1: Preparation of Comparative Compound A

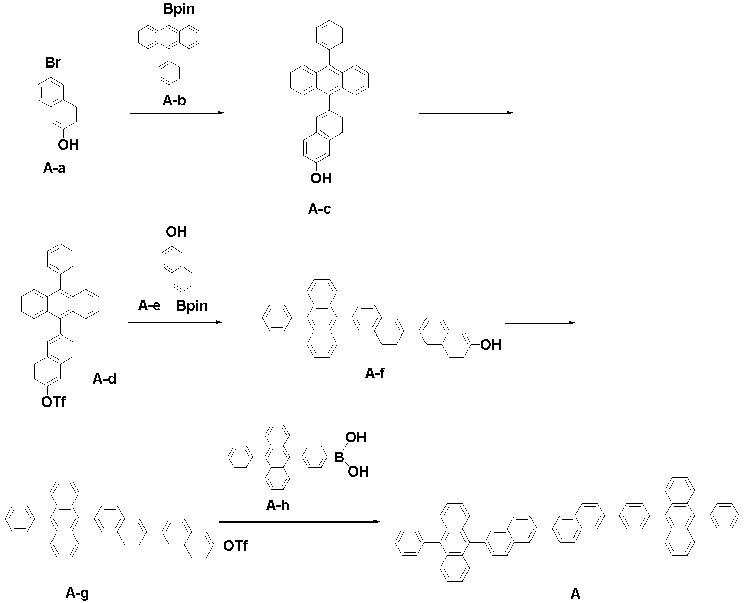

Comparative Compound A was prepared in the same manner as in Compound 1, except that in the synthesis, Compound A-a was used instead of Compound 1-a, Compound A-b instead of Compound 1-b, Compound A-c instead of Compound 1-c, Compound A-d instead of Compound 1-d, Compound A-e instead of Compound 1-e, Compound A-f instead of Compound 1-f, Compound A-g instead of Compound 1-g, and Compound A-h instead of Compound 1-h.
m/z [M+H]⁺ 835.3

### Comparative Preparation Example 2: Preparation of Comparative Compound B

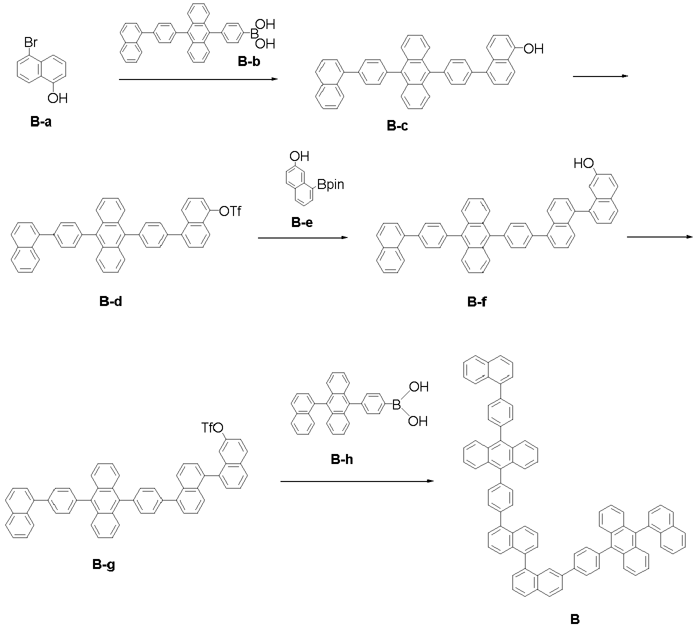

Comparative Compound B was prepared in the same manner as in Compound 1, except that in the synthesis, Compound B-a was used instead of Compound 1-a, Compound B-b instead of Compound 1-b, Compound B-c instead of Compound 1-c, Compound B-d instead of Compound 1-d, Compound B-e instead of Compound 1-e, Compound B-f instead of Compound 1-f, Compound B-g instead of Compound 1-g, and Compound B-h instead of Compound 1-h.
m/z [M+H]⁺ 1087.4

### Experimental Example 1: Solubility Measurement

It was confirmed whether each of Compounds 1 to 14, Compound A and Compound B prepared in Preparation Examples could be dissolved in cyclohexanone at a concentration of 1.3 wt% at 25°C, and the results are shown in Table 1 below. At this time, "O" means that the compound was easily dissolved in cyclohexanone, "Δ" means that the compound was dissolved in cyclohexanone at 80°C, and "X" means that the compound is not soluble in cyclohexanone.

**[Table 1]**

| Compound | Solubility (1.3 wt%) |
|---|---|
| Compound 1 | ○ |
| Compound 2 | ○ |
| Compound 3 | ○ |
| Compound 4 | ○ |
| Compound 5 | ○ |
| Compound 6 | ○ |
| Compound 7 | ○ |
| Compound 8 | ○ |
| Compound 9 | ○ |
| Compound 10 | ○ |
| Compound 11 | ○ |
| Compound 12 | ○ |
| Compound 13 | ○ |
| Compound 14 | ○ |
| Compound A | X |
| Compound B | Δ |

As can be shown in Table 1, it was confirmed that all of the compounds represented by Chemical Formula 1 could be dissolved in cyclohexanone at a concentration of 1.3 wt%, Comparative compounds A and B could not be dissolved at room temperature, and thus cannot be used as compounds constituting an organic layer when manufacturing the organic layer of an organic light emitting device by a solution process.

This is because the bond angle of the (naphthylene)-(naphthylene) linker makes a difference in solubility of the molecule. Comparative Compounds A and B having a (naphthylene)-(naphthylene) linker different from the compound represented by Chemical Formula 1 shows that the two sigma bonding orbitals of the "naphthylene" linker are unable to effectively induce a twist form in molecule, but induces only a plate-like structure, whereas the bond angle of the (naphthylene)-(naphthylene) linker of the compounds represented by Chemical Formula 1 can induce a twist form to an extent sufficient to increase solubility.

Therefore, in the case of a compound having a certain molecular weight or higher through the solubility measurement, even if it has the same moiety structure in order to increase the solubility, it can be seen that a twist form rather than a plate shape in the molecular must be effectively induced.

### Example 1

A glass substrate thinly coated with ITO (indium tin oxide) to have a thickness of 500 Å was put into distilled water in which a detergent was dissolved, and ultrasonically washed. In this case, a product manufactured by Fischer Co., was used as the detergent, and distilled water twice filtered using a filter manufactured by Millipore Co., was used as the distilled water. After the ITO was washed for 30 minutes, ultrasonic washing was conducted twice repeatedly using distilled water for 10 minutes. After the washing using distilled water was completed, ultrasonic washing was conducted using isopropyl, and acetone solvents, and drying was conducted, and then the substrate was cleaned for 5 minutes, and then transferred to a glove box.

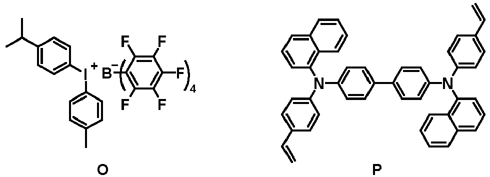

A coating composition, in which the following Compound O and the following Compound P (weight ratio of 2:8) were dissolved in 20 wt/v% cyclohexanone, was spin-coated (4000 rpm) on the ITO transparent electrode, and heat-treated (cured) at 200°C for 30 minutes to form a hole injection layer with a thickness of 400 Å.

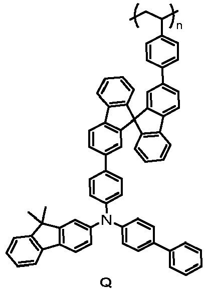

A coating composition, in which the following Compound Q (Mn: 27,900; Mw: 35,600; measured by gel permeation chromatography (GPC) on Agilent 1200 series, relative to PC standards) was dissolved in 6 wt/v% toluene, spin-coated (4000 rpm) on the hole injection layer, and heat-treated at 200°C for 30 minutes to form a hole transport layer with a thickness of 200 Å.

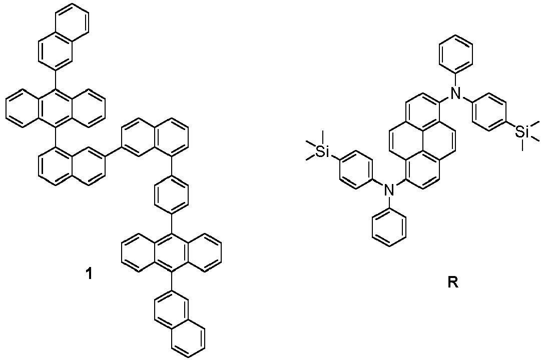

A coating composition, in which Compound 1 prepared in the previous Preparation Example 1 as a host and Compound R as a dopant of the light emitting layer (a weight ratio of 98: 2) were dissolved in 1.3 wt/v% cyclohexanone, spin-coated (4000 rpm) on the hole transport layer, and heat-treated at 180°C for 30 minutes to form a light emitting layer with a thickness of 400 Å.

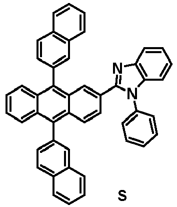

After being transferred to a vacuum depositor, the following Compound S was vacuum-deposited to a thickness of 350 Å on the light emitting layer to form an electron injection and transport layer. LiF and aluminum were sequentially deposited to have a thickness of 10 Å and 1000 Å, respectively, on the electron injection and transport layer, thereby forming a cathode.

In the above-mentioned process, the vapor deposition rate of the organic material was maintained at 0.4 to 0.7 Å/sec, the deposition rate of LiF was maintained at 0.3 Å/sec, the deposition rate of aluminum was maintained at 2 Å/sec, and the degree of vacuum during the deposition was maintained at 2*10⁻⁷ to 5*10⁻⁸ torr.

### Examples 2 to 14

An organic light emitting device was manufactured in the same manner as in Example 1, except that the compounds shown in Table 2 below were used instead of Compound 1 as a host of the light emitting layer. The compounds used in Examples 1 to 14 are summarized as follows:

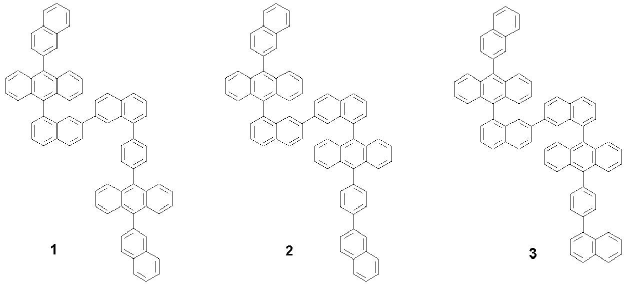

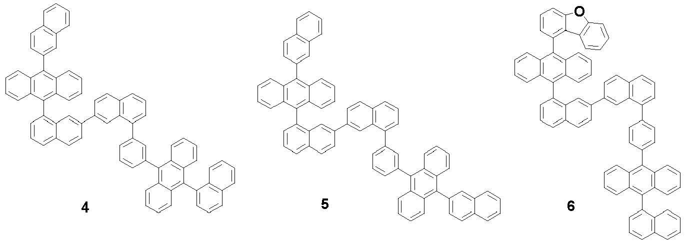

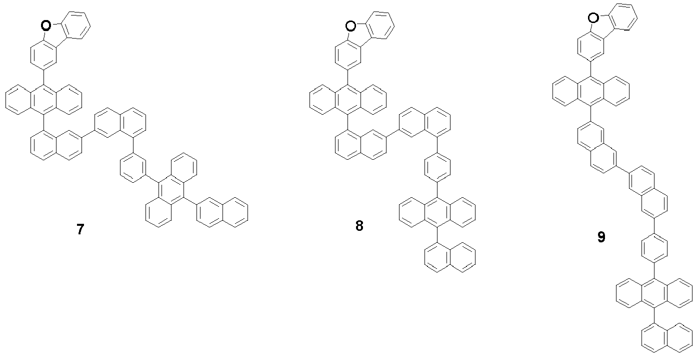

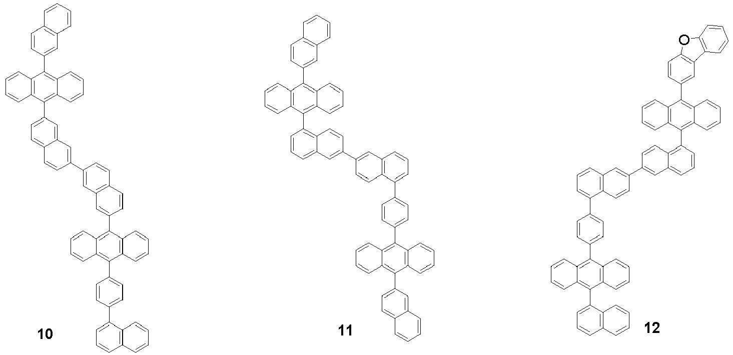

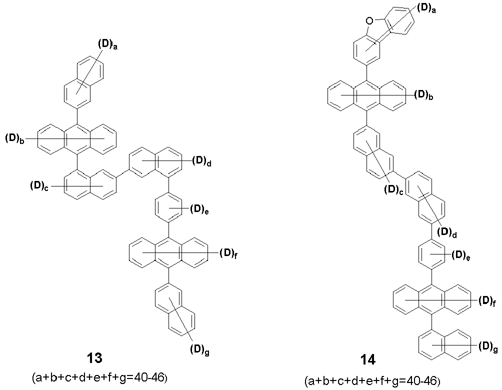

### Comparative Examples 1 and 2

An organic light emitting device was manufactured in the same manner as in Example 1, except that the compounds shown in Table 2 below were used instead of Compound 1 as a host of the light emitting layer.

### Experimental Example 2: Characteristic Evaluation of Organic Light Emitting Device

When a current is applied to the organic light emitting devices manufactured in Examples and Comparative Examples, the driving voltage, external quantum efficiency (EQE) and lifetime were measured at a current density of 10 mA/cm², and the results are shown in Table 3 below. At this time, the external quantum efficiency (EQE) was determined by "(number of photons emitted)/(number of charge carriers injected)*100", and T90 means the time required for the luminance to be reduced to 90% of the initial luminance (200 nit)

**[Table 2]**

| Category | Light emitting layer host | Driving voltage (V @10mA/cm²) | EQE (% @10mA/cm²) | Lifetime (hr) (T90 @500 nit) |
|---|---|---|---|---|
| Example 1 | Compound 1 | 6.4 | 5.53 | 621 |
| Example 2 | Compound 2 | 6.4 | 5.57 | 685 |
| Example 3 | Compound 3 | 6.6 | 5.48 | 662 |
| Example 4 | Compound 4 | 6.0 | 5.37 | 725 |
| Example 5 | Compound 5 | 6.2 | 5.32 | 679 |
| Example 6 | Compound 6 | 6.4 | 5.41 | 733 |
| Example 7 | Compound 7 | 6.1 | 5.56 | 649 |
| Example 8 | Compound 8 | 6.5 | 5.49 | 630 |
| Example 9 | Compound 9 | 6.4 | 5.43 | 680 |
| Example 10 | Compound 10 | 6.3 | 5.4 | 615 |
| Example 11 | Compound 11 | 6.5 | 5.46 | 654 |
| Example 12 | Compound 12 | 6.2 | 5.45 | 659 |
| Example 13 | Compound 13 | 6.1 | 5.59 | 748 |
| Example 14 | Compound 14 | 6.3 | 5.47 | 705 |
| Comparative Example 1 | Compound A | 6.8 | 4.05 | 321 |
| Comparative Example 2 | Compound B | 6.9 | 4.17 | 419 |

As shown in Table 2, it is confirmed that the organic light emitting device including the compound of the present disclosure as a host in the light emitting layer exhibits improved efficiency and lifetime while exhibiting a voltage at a level comparable to that of the devices of Comparative Examples.

Specifically, the organic light emitting device of Examples in which the compound represented by Chemical Formula 1 was used as a host of the light emitting layer was remarkably improved in efficiency and lifetime as compared to the organic light emitting devices of Comparative Examples 1 and 2 in which Comparative Compounds A and B were respectively used as hosts of the light emitting layer. This is judged to be because the material stability was improved due to the structural characteristics possessed by the compound represented by Chemical Formula 1.

Therefore, when employing the compound represented by Chemical Formula 1 as a host material of an organic light emitting device, it can be seen that the external quantum efficiency and lifetime characteristics of the organic light emitting device can be simultaneously improved.

**[Description of Symbols]**

| | | | |
|---|---|---|---|
| 1: | substrate | 2: | anode |
| 3: | light emitting layer | 4: | cathode |
| 5: | hole injection layer | 6: | hole transport layer |
| 7: | electron injection and transport layer | | |

## Claims

1. A compound represented by the following Chemical Formula 1: 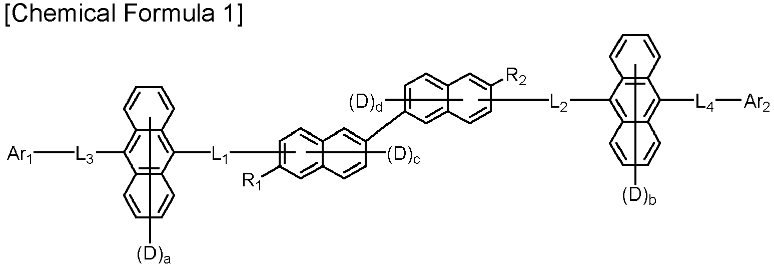 wherein, in Chemical Formula 1,
D means deuterium,
R₁ and R₂ each independently represent hydrogen or deuterium;
L₁ to L₄ each independently represent a single bond; or a substituted or unsubstituted C₆₋₆₀ arylene,
Ar₁ and Ar₂ each independently represent a substituted or unsubstituted C₆₋₆₀ aryl; or a substituted or unsubstituted C₂₋₆₀ heteroaryl containing one or more heteroatoms of N, O and S,
a and b are each independently an integer of 0 to 8, and
c and d are each independently an integer of 0 to 5.

2. The compound of claim 1, wherein:
both R₁ and R₂ are hydrogen; or deuterium.

3. The compound of claim 1, wherein:
L₁ to L₄ each independently represent a single bond; 1,2-phenylene unsubstituted or substituted with 1 to 4 deuterium atoms; 1,3-phenylene unsubstituted or substituted with 1 to 4 deuterium atoms; or 1,4-phenylene unsubstituted or substituted with 1 to 4 deuterium atoms.

4. The compound of claim 1, wherein:
both L₁ and L₂ are a single bond; or
one of L₁ and L₂ is a single bond, and the remaining one is phenylene unsubstituted or substituted with 1 to 4 deuterium atoms.

5. The compound of claim 1, wherein:
both L₃ and L₄ are a single bond; or
one of L₃ and L₄ is a single bond, and the remaining one is phenylene unsubstituted or substituted with 1 to 4 deuterium atoms.

6. The compound of claim 1, wherein:
Ar₁ and Ar₂ each independently represent phenyl, biphenylyl, naphthyl, phenanthryl, dibenzofuranyl, dibenzothiophenyl, carbazolyl, or phenylcarbazolyl,
wherein Ar₁ and Ar₂ are unsubstituted or substituted with one or more deuterium atoms.

7. The compound of claim 1, wherein:
a+b+c+d is 0, or 10 or more.

8. The compound of claim 1, wherein:
the compound is represented by any one of the following Chemical Formulas 1-1 to 1-3: 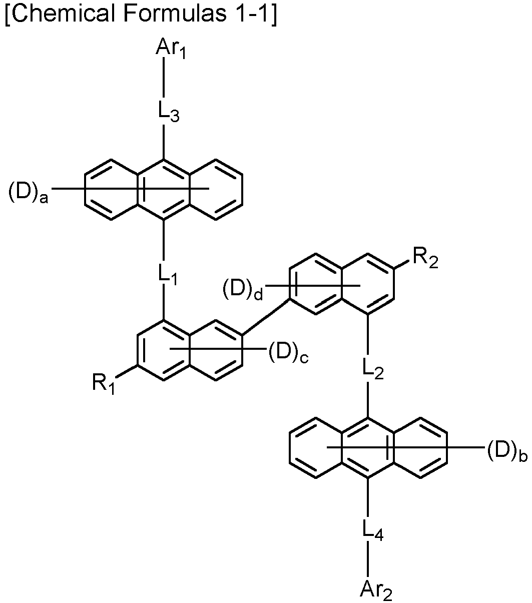 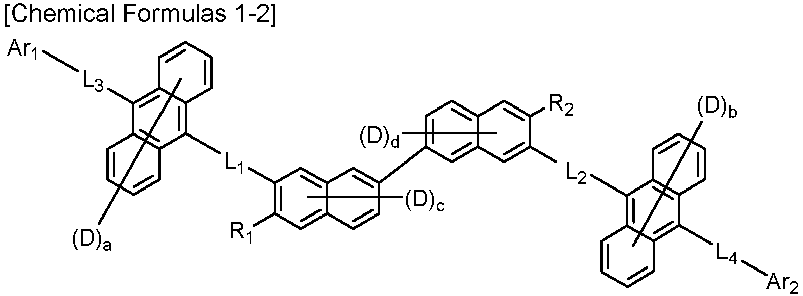 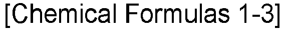 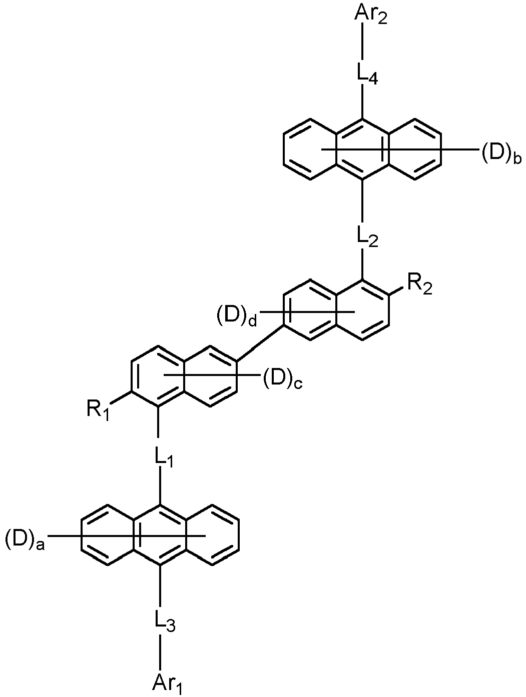 wherein, in Chemical Formulas 1-1 to 1-3,
R₁, R₂, L₁ to L₄, Ar₁, Ar₂ and a to d are as defined in claim 1.

9. The compound of claim 1, wherein:
the deuterium substitution rate of the compound is 0%, or 80% or more.

10. The compound of claim 1, wherein:
the compound has a molecular weight of 800 g/mol or more.

11. The compound of claim 1, wherein:
the compound is any one selected from the group consisting of compounds represented by the following Chemical Formulas and their deuterated structures:

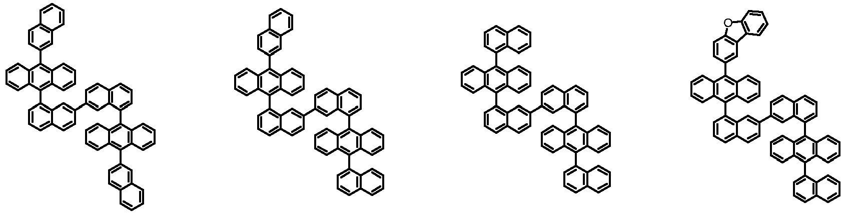

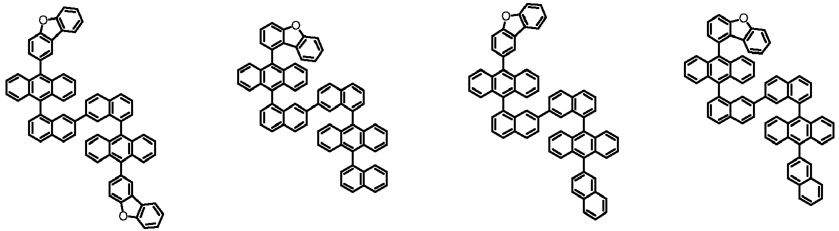

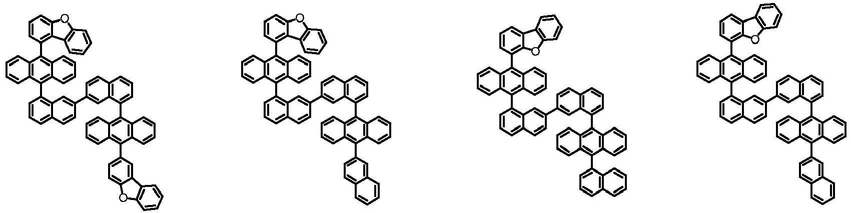

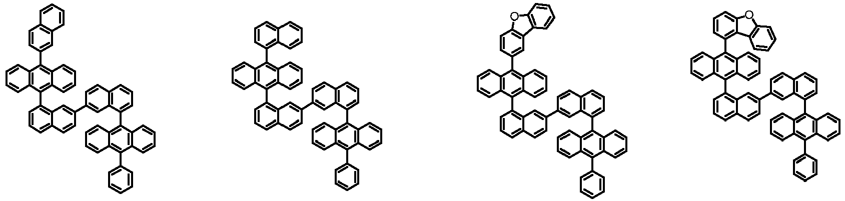

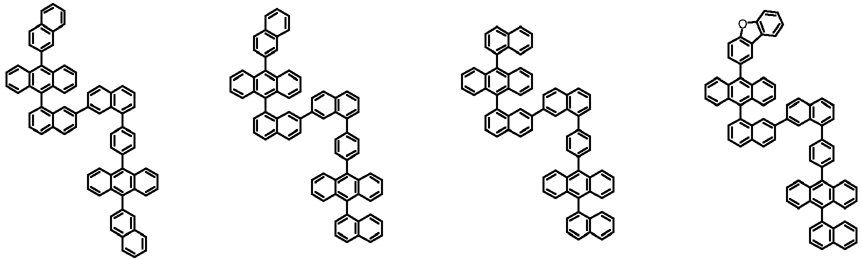

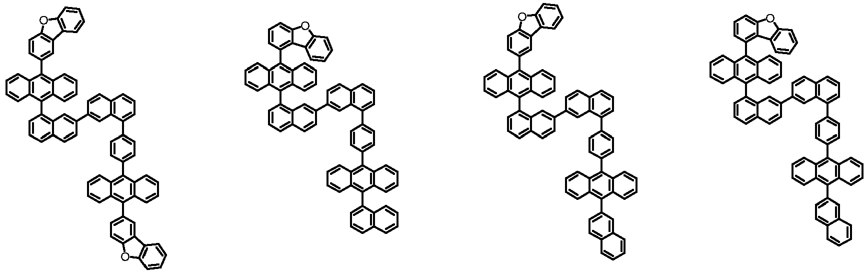

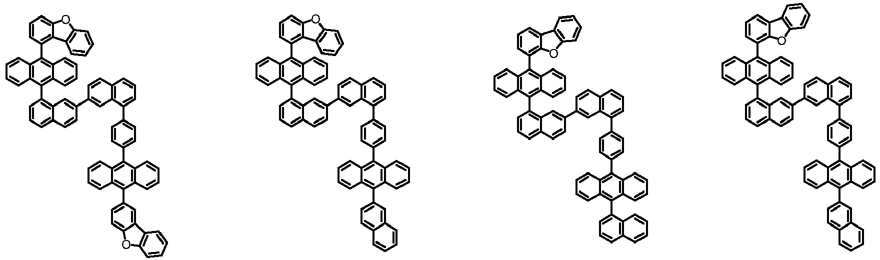

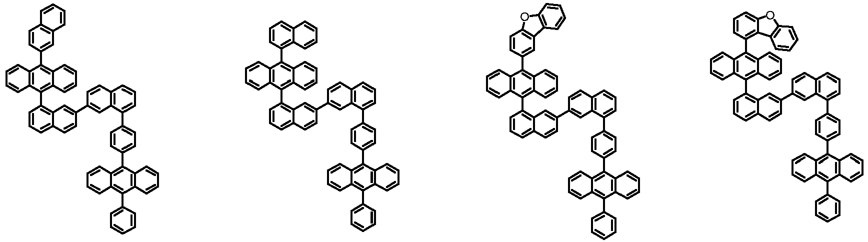

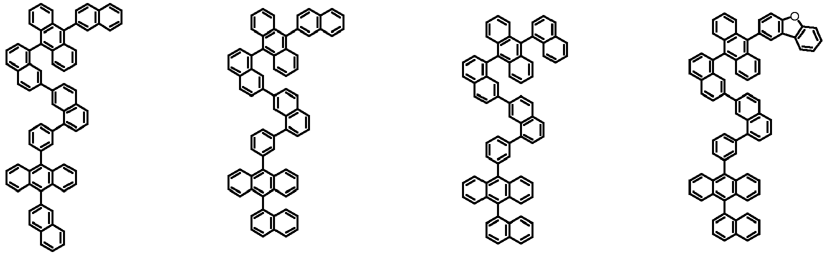

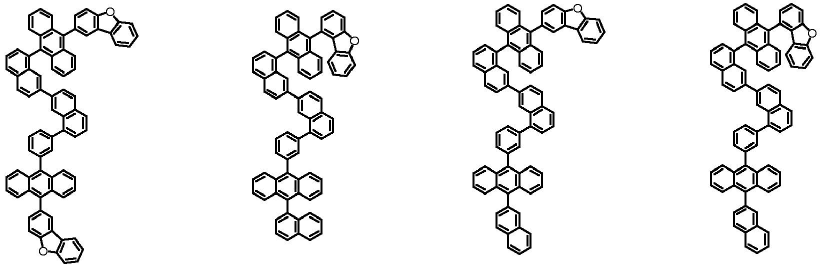

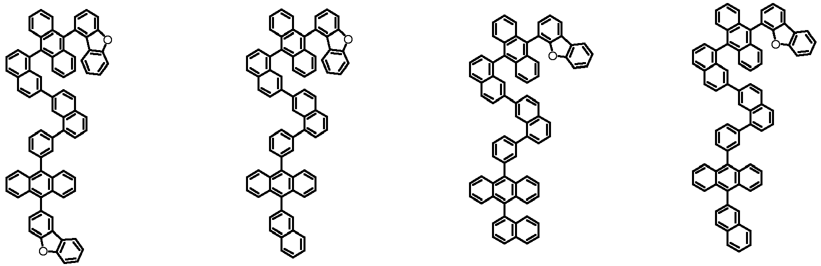

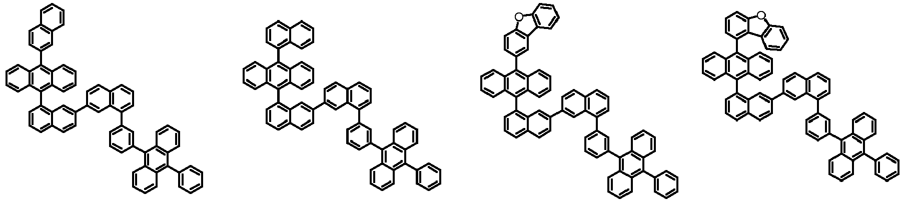

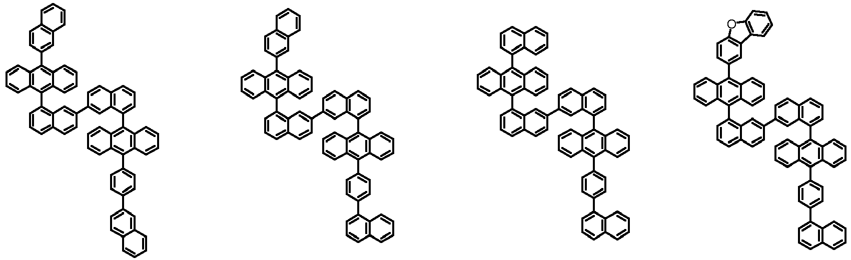

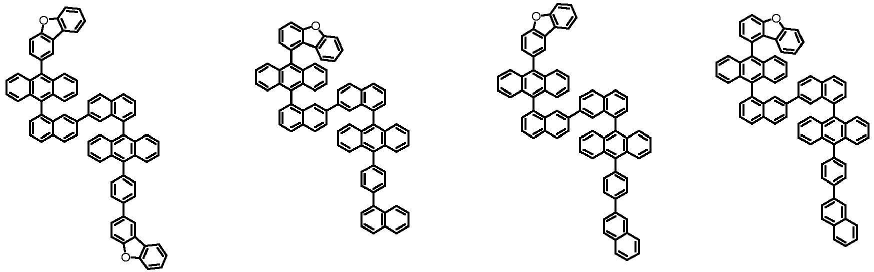

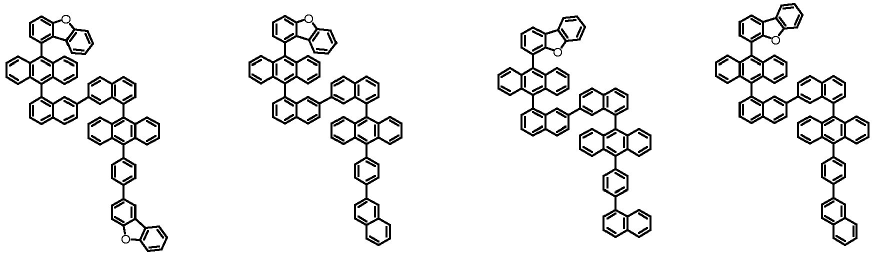

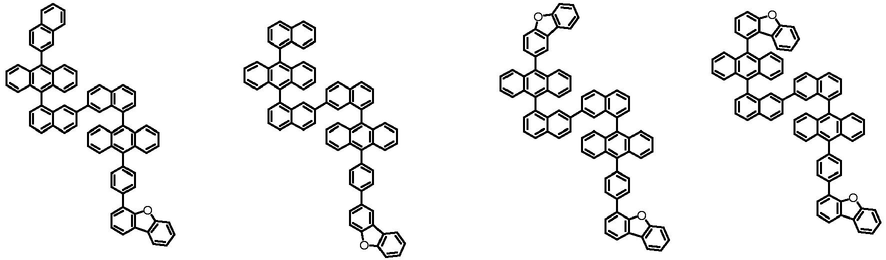

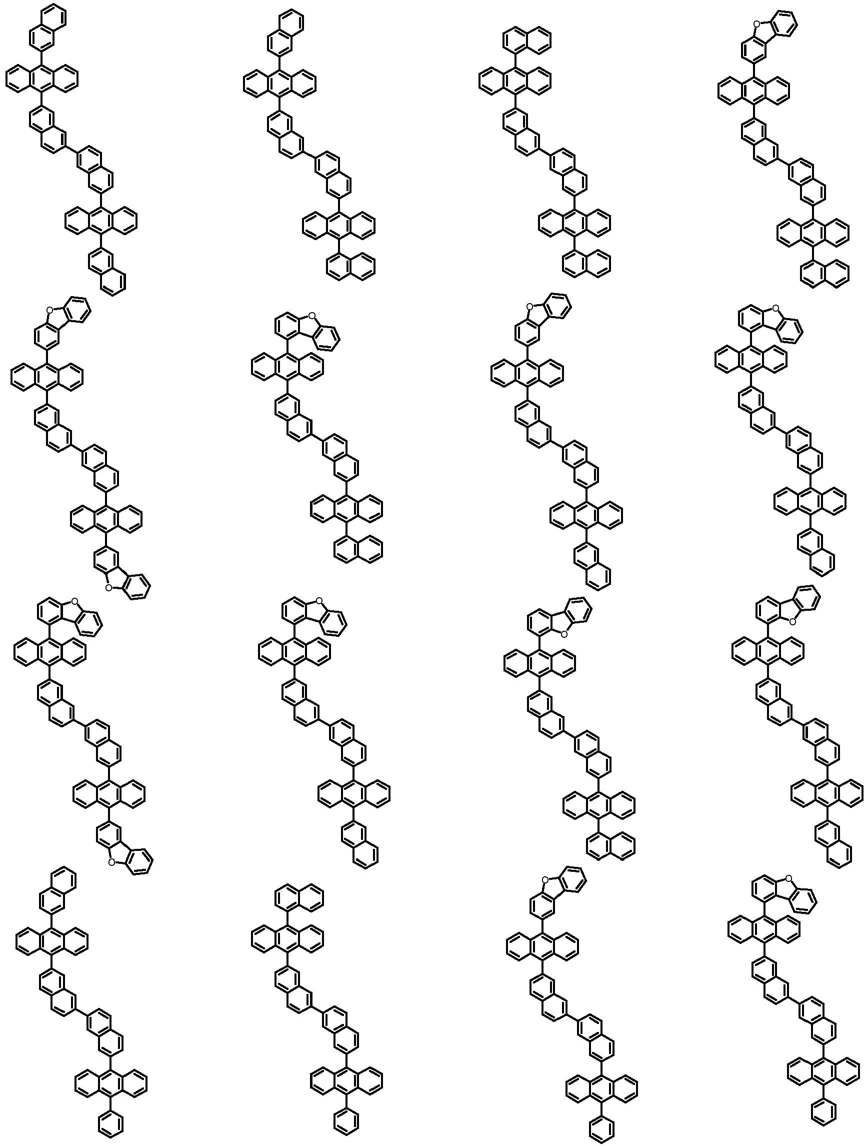

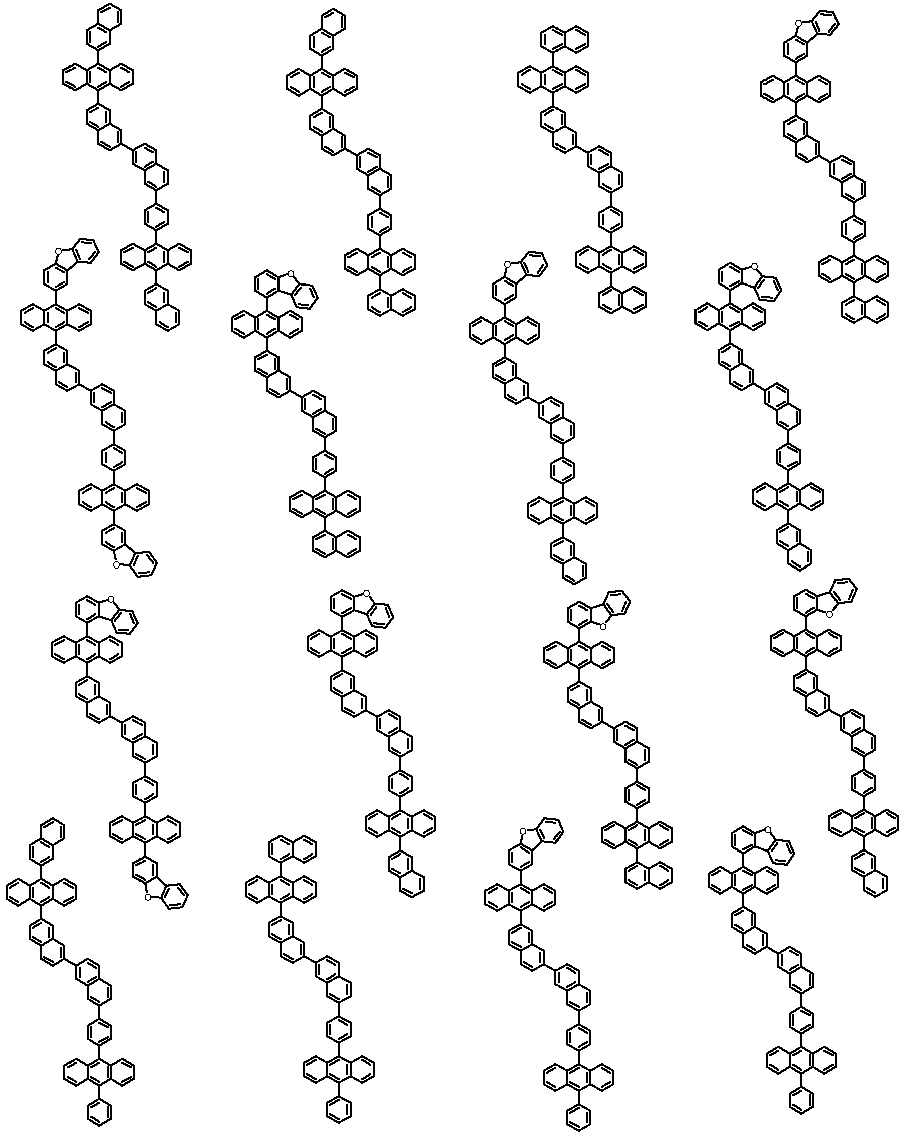

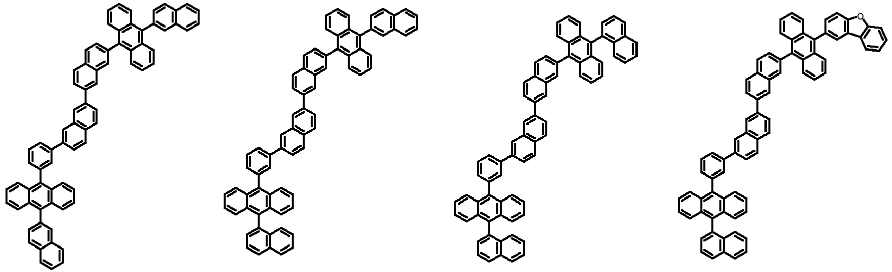

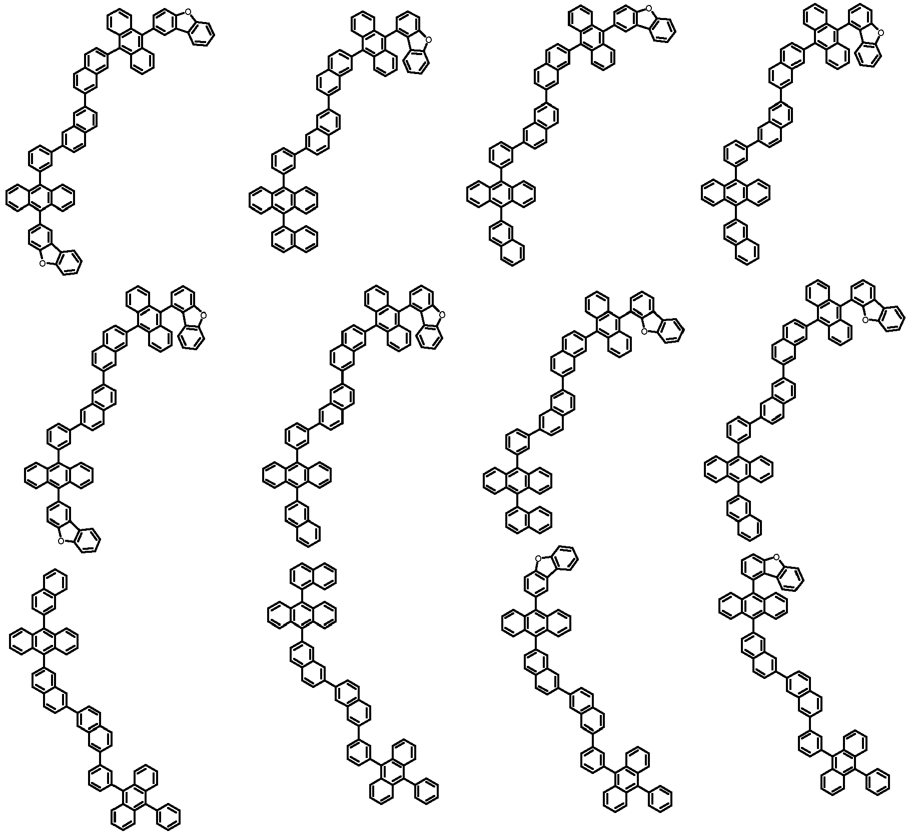

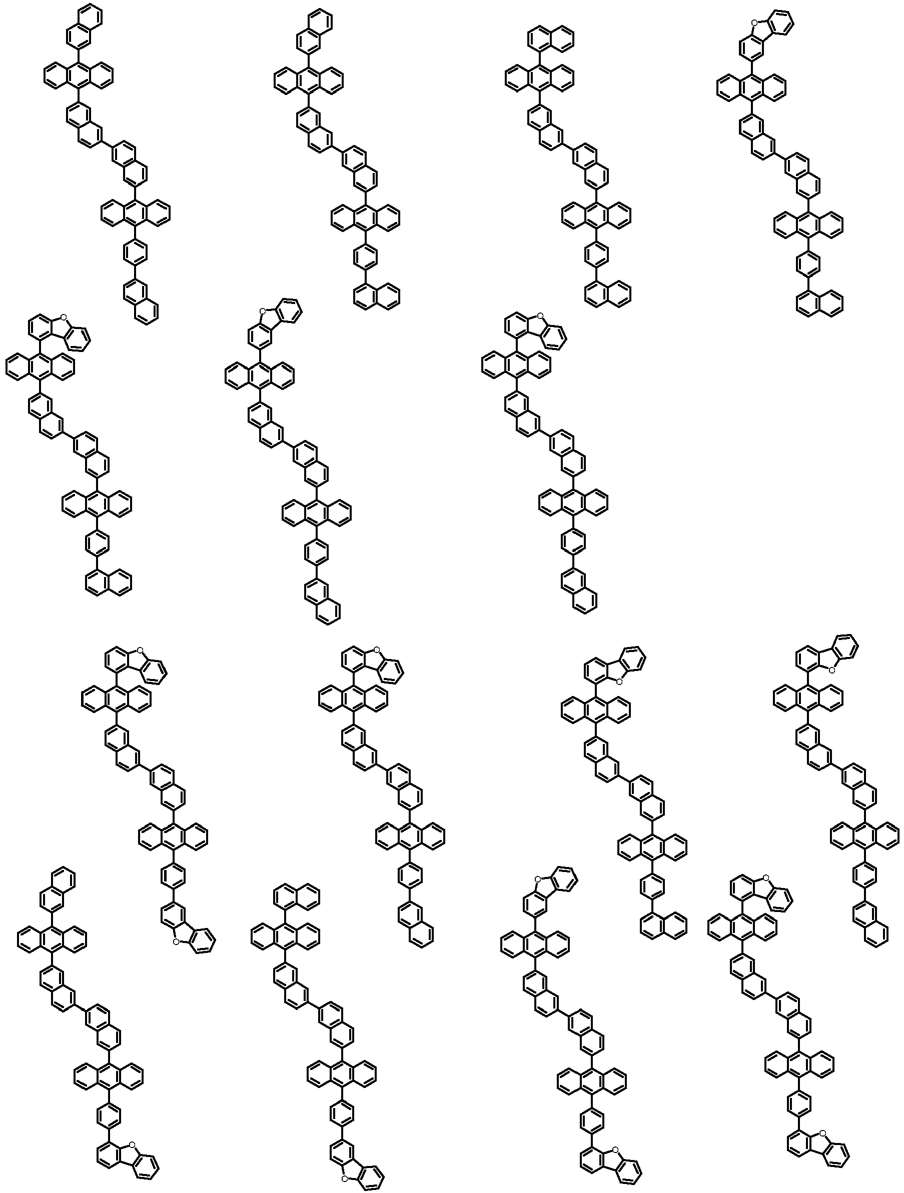

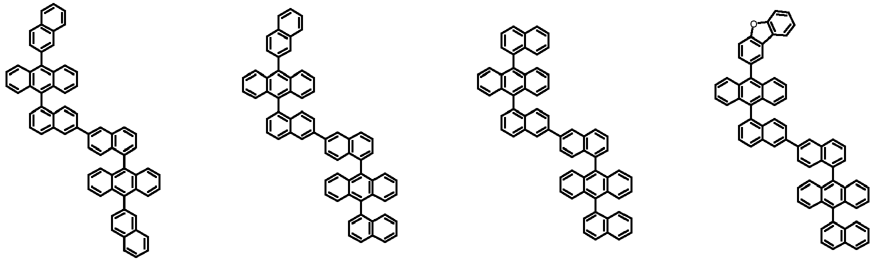

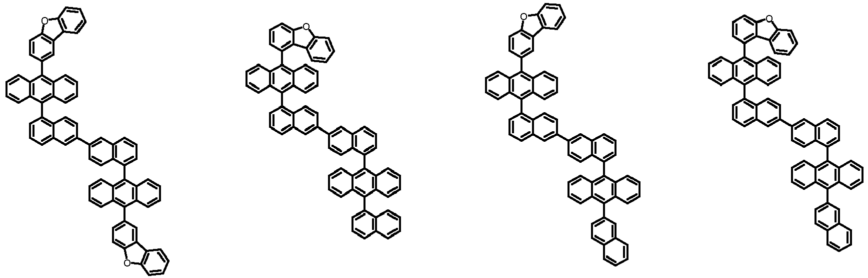

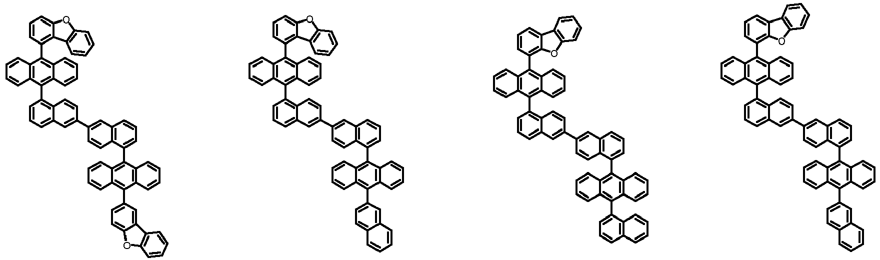

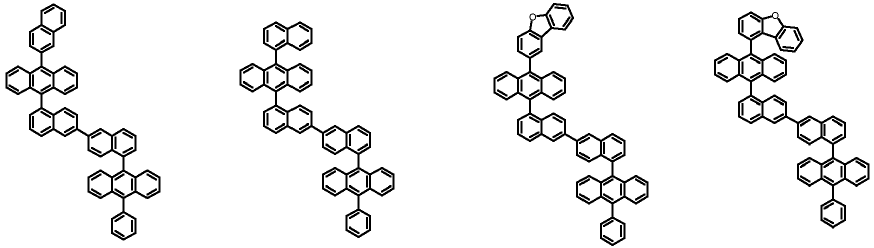

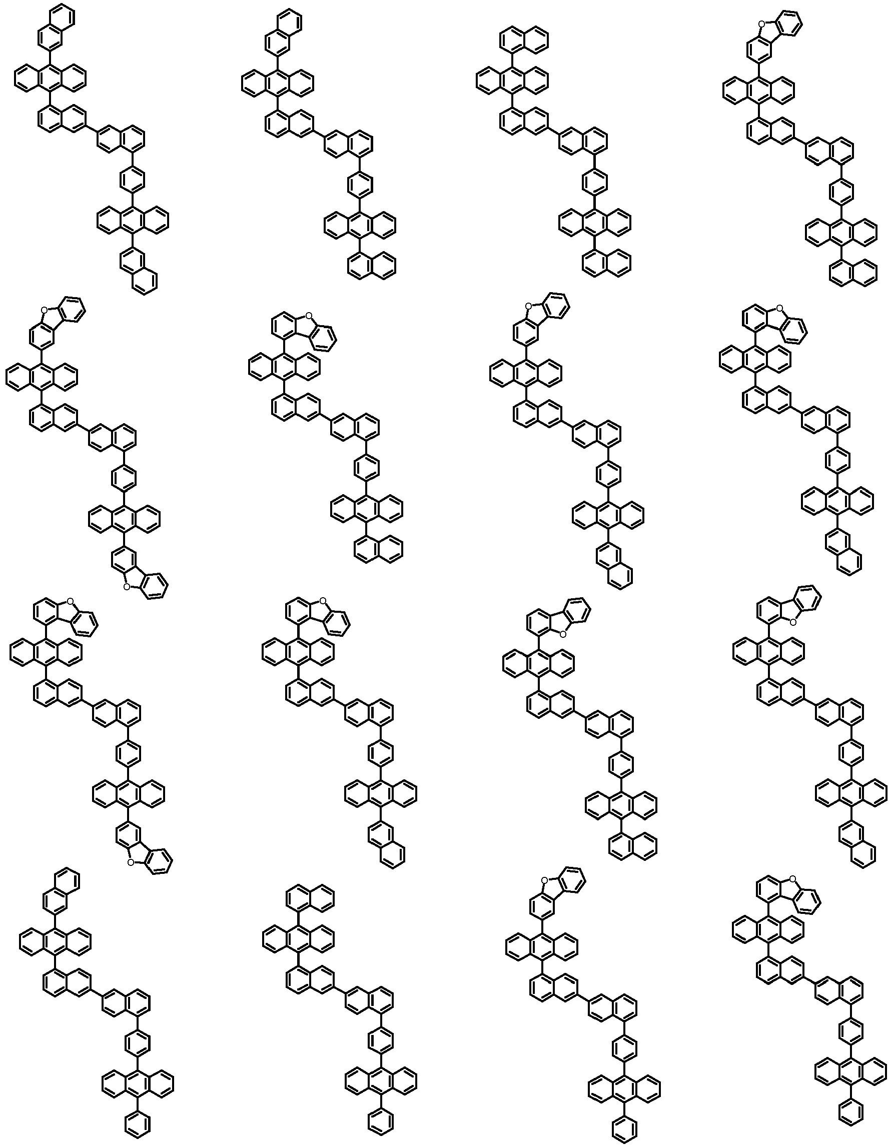

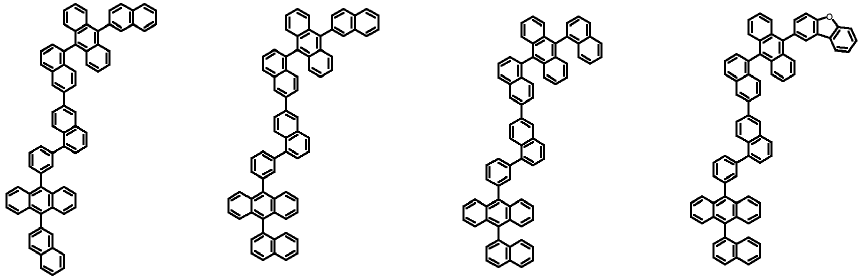

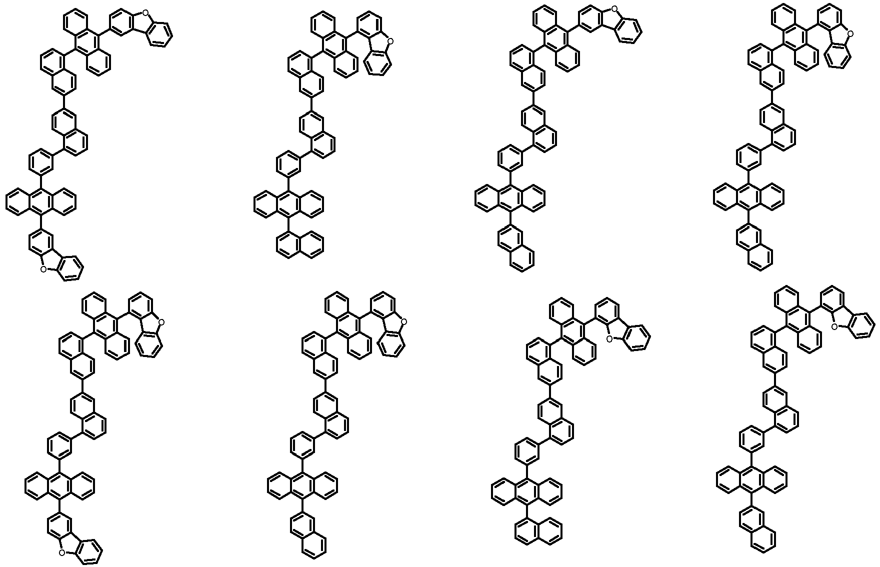

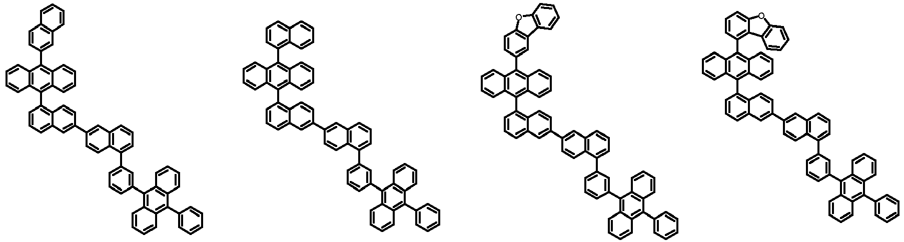

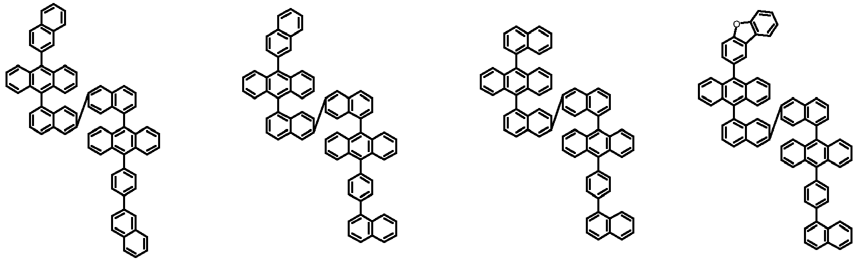

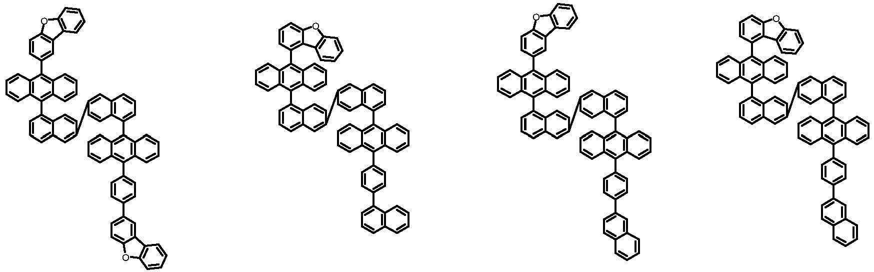

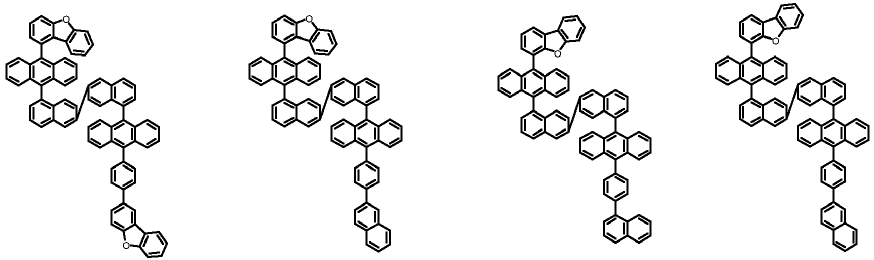

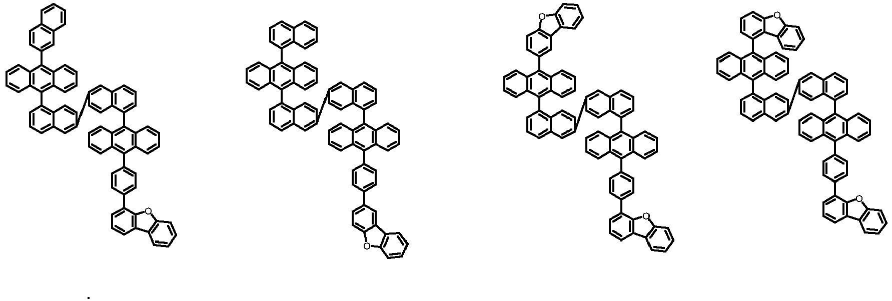

12. An organic light emitting device comprising: a first electrode; a second electrode that is provided opposite to the first electrode; and one or more organic material layers that are provided between the first electrode and the second electrode, wherein the one or more layers of the organic material layers comprise the compound as defined in any one of claims 1 to 11.

13. An organic light emitting device of claim 12, wherein:
the organic material layer comprising the compound is a light emitting layer.
